# EUROPEAN PATENT APPLICATION

(11) **EP 2 567 957 A1**
(43) Date of publication of application: **13.03.2013**
(21) Application number: 12195826.8
(22) Date of filing: 29.09.2010
(51) Int. Cl.: C07D 405/06, C07D 405/14, C07D 413/06, C07D 413/14, C07D 417/06, C07D 417/14, A61K 31/357, A61P 3/06

(54) **Compounds for the treatment of dyslipidemia and related diseases**

(30) Priority: 01.10.2009 IN MU22922009
(62) Divisional of application: 10810948.9
(71) Applicant: CADILA HEALTHCARE LIMITED, Ahmedebad 380 015, Gujarat (IN)
(72) Inventor: Pingali, Harikishore, 380 015 Gujarat (IN); Kalapatapu, V., V., M., Sairam, 380 015 Gujarat (IN); Makadia, Pankaj, 380 015 Gujarat (IN); Jain, Mukul, R., 380 015 Gujarat (IN)
(74) Representative: Viering, Jentschura & Partner

(57) **Abstract**

The present invention relates to compounds of the general formula (I), their tautomeric forms, their stereoisomers, their pharmaceutically acceptable salts, pharmaceutical compositions containing them, methods for their preparation, use of these compounds in medicine and the intermediates involved in their preparation.

## Description

### FIELD OF INVENTION

The present invention relates to compounds of the general formula (I), their tautomeric forms, their stereoisomers, their pharmaceutically acceptable salts, pharmaceutical compositions containing them, methods for their preparation, use of these compounds in medicine and the intermediates involved in their preparation

The present invention is directed to antagonists of PCSK9 which can be used to treat the diseases mediated through PCSK9 enzyme e.g. dyslipidemia. The compounds of the present invention also lower LDL-c.

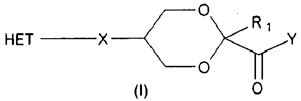

### BACKGROUND OF THE INVENTION

Higher LDL cholesterol levels in the plasma increase cardiovascular risk and reduction in the levels of LDL would decrease CVD risk by a comparable percentage (PNAS, 2009, 106, 9546-9547). Clearance of LDL cholesterol from plasma is through the action of LDL receptors in the liver and LDL receptors are cell surface glycoproteins that bind to apoliporpotein B100 (apoB100) on LDL particles with high affinity and mediate their endocytic uptake (Journal of Biological Chemistry, 2009, 284, 10561-10570). Defect in hepatic cholesterol clearance and elevated levels of plasma LDL cholesterol that result from the mutations cause familial hypercholesterolemia. Such mutations are identified in the human LDL receptor and later in apolipoprotein-B (Nature Structural and Molecular Biology, 2007, 14, 413-419). Recently, mutations within the proprotein convertase subtilisin/kexin type9 (PCSK9) gene were found to represent a third class of mutations associated with autosomal dominant hypercholesterolemia (ADH). Abifadel *et al* in 2003 discovered PCSK9 as the third gene involved in autosomal dominant hypercholesterolaemia (ADH) (Nature Genetics, 2003, 34, 154-156. Trends in Biochemical Sciences, 2008, 33, 426-434). Several missense mutations (S127R, D129G, F216L, D374H, D374Y) are associated with hypercholesterolemia and premature atherosclerosis (J Lipid Res. 2008, 49, 1333-1343). Loss-of-function mutations (R46L, L253F, A433T) lead to elevated receptor abundance, enhancing clearance of LDL cholesterol from the circulation and reducing cardiovascular risk (Nature Structural and Molecular Biology, 2007, 14, 413-419).

PCSK9 belongs to the subtilisin family of serine proteases and its protein structure consists of a prodomain, catalytic domain, and cysteine/histidine rich C-terminal domain (Structure, 2007, 15, 545-552). Unlike other proprotein convertases, wherein the prodomain is further proteolytically processed to activate the serine protease, the prodomain of secreted PCSK9 remains intact and tightly bound. Within endoplasmic reticulum PCSK9 undergoes autocatalytic process which results in release of ~14 kDa prodomain that remains associated with the catalytic/C-terminal domains, wherein the prodomain serves as both a folding chaperon and as an inhibitor of enzymatic activity (Journal of Biological Chemistry, 2009, 284, 10561-10570).
It is well documented that epidermal growth factor-like repeat A (EGF-A) of LDLR interacts with PCSK9 mainly with residues 367-381. This EGF-A interaction site is located >20 Å from the catalytic site of PCSK9. Once EGF-A and PCSK9 interacts they form a PCSK9-LDLR complex that enters endosomal pathway and hence LDLR recycling is prevented leading to LDLR degradation. Detailed molecular mechanisms explaining the association of LDLR and PCSK9 and LDLR degradation is not very clear (Drug News Perspectives, 2008, 21, 323-330). Because of inhibition of LDLR recycling, number of LDL receptors on the cell surface are decreased and this increases plasma LDL levels (PNAS, 2009, 106, 9546-9547).

Various approaches for inhibiting PCSK9 are reported, including gene silencing by siRNA or antisense oligonucleotides, mAb disrupting protein-protein interactions or by peptides; all the above-mentioned strategies have shown lowering of LDL cholesterol which may be effective therapy for treating hypercholesterolemia (Biochemical Journal, 2009, 419, 577-584; PNAS, 2008, 105, 11915-11920; Journal of Lipid Research, 2007, 48, 763-767; PNAS, 2009, 106, 9820-9825).
There are many reports mentioning the advantage of having a small molecule inhibitor for PCSK9 (Drug News Perspect 21 (6), July/August 2008, 323-330; PNAS, 106 (24), 9546-9547, 2009; Curr Atheroscler Rep (2010) 12:308-315 etc.). Since then it has been the endeavor of many to discover small molecule inhibitors of PCSK9. Small molecules targeting PCSK9 looked difficult because, LDL-R degradation of PCSK9 is a protein-protein interaction and the process is independent of PCSK9 proteolytic activity (Lou, K.-J. *SciBX* **2**(22); doi:10.1038/scibx.2009.895 and respective references therein). In the light of above-mentioned reports, we have hypothesized that small molecule binding to catalytic site of PCSK9 could allosterically effect LDL-R binding to PCSK9. Using in silico docking studies we have designed compounds that can bind to PCSK9 catalytic site. Such molecules as mentioned in the examples have shown SPR binding, in vitro (ELISA) potency and in vivo efficacy as a proof of concept.

### SUMMARY OF THE INVENTION

The main objective of the present invention is to provide compounds of formula (1) or their pharmaceutically acceptable salts which are modulators of the PCSK9 enzyme.

In an embodiment of the present invention is provided a process for the preparation of the compounds of the present invention.

In a further embodiment of the present invention is provided process for treatment of diseases mediated by the PCSK9 enzyme by providing therapeutically effective amount of the compounds of formula (I) or their pharmaceutically acceptable salts or their suitable pharmaceutical compositions.
In an embodiment is provided a method of identifying small molecules as inhibitors of PCSK9.

The above and other embodiments are described hereinafter.

### DESCRIPTION OF THE FIGURE

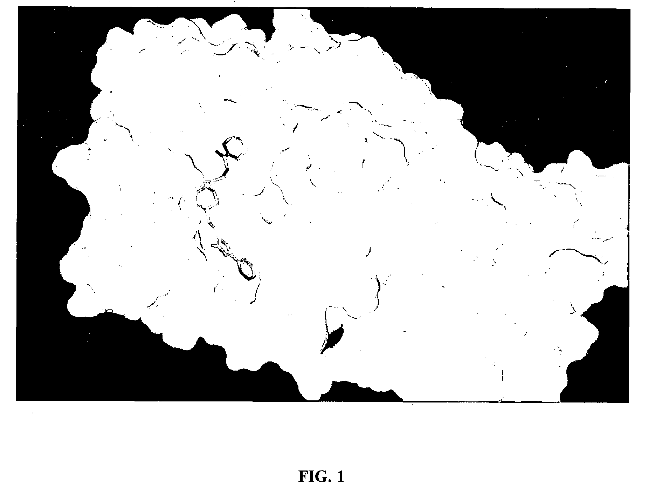

Figure 1 is a depiction of three dimensional model of PCSK9 docked pose with Example 47. Ligand binding mode is shown with electrostatic surface on the PCSK9 protein.

### DETAILED DESCRIPTION OF THE INVENTION

Accordingly, the present invention relates to compounds of the general formula (I), their tautomeric forms, their stereoisomers, regioisomers, their pharmaceutically acceptable salts, and pharmaceutical compositions containing them wherein

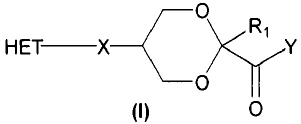

'HET' represents an optionally substituted heteroaryl or a heterocyclic group;

When the groups 'HET' is substituted, the substituents represents one or more groups selected from hydrogen or optionally substituted groups selected from (C1-C6) linear or branched alkyl, aryl, heteroaryl, cycloalkyl or a heterocyclyl group In a preferred embodiment, substitution on 'HET' are selected from (C1-C6) linear or branched alkyl, aryl or heteroaryl groups;

'X' represents the groups selected from (CH₂)ₙ, O(CH₂)ₙ, S(CH₂)ₙ, SO(CH₂)ₙ, SO₂(CH₂)ₙ or NR₂(CH₂)ₙ wherein R₂ represents H, C₍₁₋₆₎ linear or branched alkyl or a cycloalkyl group, wherein either the alkyl or cycloalkyl group may be further substituted with one or more groups as described hereinafter and n = 0 to 3;

'R₁' represents linear or branched C₍₁₋₆₎ alkyl, or suitable groups selected from cycloalkyl, aryl, groups,;

In one embodiment, 'Y' represents NR₃R₄ wherein R₃ represents C₍₁₋₆₎ linear or branched alkyl, cycloalkyl, cycloalkylalkyl, aryl, heteroaryl, heterocyclyl, arylalkyl, heteroarylalkyl, heterocyclylalkyl, hydroxy, alkoxy, cycloalkoxy, cycloalkylalkoxy, aryloxy, arylalkoxy, heteroaryloxy, heteroarylalkoxy, heterocyclyloxy or heterocyclylalkoxy groups, each of which may be further substituted with one or more groups from those described hereinafter and R₄ represents H, C₍₁₋₆₎ linear or branched alkyl, cycloalkyl, cycloalkylalkyl, aryl, heteroaryl, heterocyclyl, arylalkyl, heteroarylalkyl or heterocyclylalkyl groups, each of which may be further substituted with one or more groups from those described hereinafter;
In another embodiment, 'Y' represents the groups 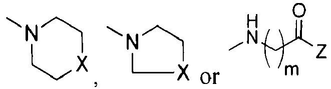 wherein 'P' represents CH₂, S, SO, SO₂, O, CO or NR₅ wherein R₅ represents H, C₍₁₋₆₎ linear or branched alkyl or cyclo alkyl groups; 'm' represents integers from 1 to 3;
and 'Z' represents either NR₃R₄ or the groups 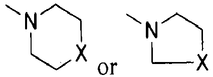 wherein R₃, R₄ and P are as defined earlier;

When any of the groups mentioned in the specification is substituted, the substituents at each occurrence may be independently selected from hydroxyl, oxo, halo, thiol, nitro, amino, cyano, formyl, or substituted or unsubstituted groups selected from amidino, alkyl, haloalkyl, perhaloalkyl, alkoxy, haloalkoxy, perhaloalkoxy, alkenyl, alkynyl, cycloalkyl, cycloalkenyl, bicycloalkyl, bicycloalkenyl, alkoxy, alkenoxy, cycloalkoxy, aryl, aryloxy, aralkyl, aralkoxy, heterocylyl, heteroaryl, heterocyclylalkyl, heteroaralkyl, heteroaryloxy, heteroaralkoxy, heterocycloxy, heterocyclylalkoxy, heterocyclylalkoxyacyl, acyl, acyloxy, acylamino, monosubstituted or disubstituted amino, arylamino, aralkylamino, carboxylic acid and its derivatives such as esters and amides, carbonylamino, hydroxyalkyl, aminoalkyl, alkoxyalkyl, aryloxyalkyl, aralkoxyalkyl, alkylthio, thioalkyl, cycloalkylthio, arylthio, heterocyclylthio, heteroarylthio, alkylsulfinyl, cycloalkylsulfinyl, arylsulfinyl, heterocyclylsulfinyl, heteroarylsulfinyl, alkylsulfonyl, cycloalkylsulfonyl, arylsulfonyl, heterocyclylsulfonyl, heteroarylsulfonyl, alkylsulfonylamino, cycloalkylsulfonylamino, arylsulfonylamino, heterocyclylsulfonylamino, heteroarylsulfonylamino, alkylsulfonyloxy, cycloalkylsulfonyloxy, arylsulfonyloxy, heterocyclylsulfonyloxy, heteroarylsulfonyloxy, alkoxycarbonylamino, aryloxycarbonylamino, aralkyloxycarbonylamino, aminocarbonylamino, alkylaminocarbonylamino, alkoxyamino, hydroxyl amino, sulfinyl derivatives, sulfonyl derivatives, sulfonic acid and its derivatives;

When the substituents on 'HET' is further substituted, the substituents are selected from halogen, alkyl, haloalkyl, alkoxy, sulfanyl derivative, sulfinyl derivatives, sulfonyl derivatives, sulfonyloxy groups;

The term "heterocyclyl" or "heterocyclic" group used either alone or in combination with other radicals, is selected from suitable saturated, partially saturated or unsaturated aromatic or non aromatic mono, bi or tricyclic radicals, containing one or more heteroatoms selected from nitrogen, sulfur and oxygen. In a preferred embodiment the heterocyclyl groups are selected from aziridinyl, azetidinyl, pyrrolidinyl, imidazolidinyl, piperidinyl, piperazinyl, 2-oxopiperidinyl, 4-oxopiperidinyl, 2-oxopiperazinyl, 3-oxopiperazinyl, morpholinyl, thiomorpholinyl, 2-oxomorpholinyl, azepinyl, diazepinyl, oxapinyl, thiazepinyl, oxazolidinyl, thiazolidinyl, dihydrothiophene, dihydropyran, dihydrofuran, dihydrothiazole, benzopyranyl, benzopyranonyl, benzodihydrofuranyl, benzodihydrothienyl, pyrazolopyrimidonyl, azaquinazolinoyl, thienopyrimidonyl, quinazolonyl, pyrimidonyl, benzoxazinyl, benzoxazinonyl, benzothiazinyl, benzothiazinonyl, thieno piperidinyl, and the like;

The term "heteroaryl" or "heteroaromatic" group used either alone or in combination with other radicals, is selected from suitable single or fused mono, bi or tricyclic aromatic heterocyclic radicals containing one or more hetero atoms selected from O, N or S. In a preferred embodiment the heteroaromatic groups are selected from pyridyl, thienyl, furyl, pyrrolyl, oxazolyl, thiazolyl, isothiazolyl, imidazolyl, isoxazolyl, oxadiazolyl, thiadiazolyl, triazolyl, tetrazolyl, benzofuranyl, benzothienyl, indolinyl, indolyl, azaindolyl, azaindolinyl, pyrazolopyrimidinyl, azaquinazolinyl, pyridofuranyl, pyridothienyl, thienopyrimidyl, quinolinyl, pyrimidinyl, pyrazolyl, quinazolinyl, pyridazinyl, triazinyl, benzimidazolyl, benzotriazolyl, phthalazynil, naphthylidinyl, purinyl, carbazolyl, phenothiazinyl, phenoxazinyl, benzoxazolyl, benzothiazolyl and the like.

Preferred 'HET' are selected from aziridinyl, azetidinyl, pyrrolidinyl, imidazolidinyl, piperidinyl, piperazinyl, 2-oxopiperidinyl, 4-oxopiperidinyl, 2-oxopiperazinyl, 3-oxopiperazinyl, morpholinyl, thiomorpholinyl, 2-oxomorpholinyl, azepinyl, diazepinyl, oxapinyl, thiazepinyl, oxazolidinyl, thiazolidinyl, dihydrothiophene, dihydropyran, dihydrofuran, dihydrothiazole, benzopyranyl, benzopyranonyl, benzodihydrofuranyl, benzodihydrothienyl, pyrazolopyrimidonyl, azaquinazolinoyl, thienopyrimidonyl, quinazolonyl, pyrimidonyl, benzoxazinyl, benzoxazinonyl, benzothiazinyl, benzothiazinonyl, thieno piperidinyl, pyridyl, thienyl, furyl, pyrrolyl, oxazolyl, thiazolyl, isothiazolyl, imidazolyl, isoxazolyl, oxadiazolyl, thiadiazolyl, triazolyl, tetrazolyl, benzofuranyl, benzothienyl, indolinyl, indolyl, azaindolyl, azaindolinyl, pyrazolopyrimidinyl, azaquinazolinyl, pyridofuranyl, pyridothienyl, thienopyrimidyl, quinolinyl, pyrimidinyl, pyrazolyl, quinazolinyl, pyridazinyl, triazinyl, benzimidazolyl, benzotriazolyl, phthalazynil, naphthylidinyl, purinyl, carbazolyl, phenothiazinyl, phenoxazinyl, benzoxazolyl, benzothiazolyl groups.

The various groups, radicals and substituents used anywhere in the specification are described in the following paragraphs.
- the "alkyl" group used either alone or in combination with other radicals, denotes a linear or branched radical containing one to six carbons, selected from methyl, ethyl, n-propyl, *iso*-propyl, n-butyl, *sec*-butyl, *tert*-butyl, amyl, t-amyl, n-pentyl, n-hexyl, and the like;
- the "alkenyl" group used either alone or in combination with other radicals, is selected from a radical containing from two to six carbons, more preferably groups selected from vinyl, allyl, 2-butenyl, 3-butenyl, 2-pentenyl, 3-pentenyl, 4-pentenyl, 2-hexenyl, 3-hexenyl, 4-hexenyl and the like; the "alkenyl" group includes dienes and trienes of straight and branched chains;
- the "alkynyl" group used either alone or in combination with other radicals, is selected from a linear or branched radical containing two to six carbon atoms, more preferably thynyl, 1-propynyl, 2-propynyl, 1-butynyl, 2-butynyl, 3-butynyl, 1-pentynyl, 2-pentynyl, 3-pentynyl, 4-pentynyl, 1-hexynyl, and the like. The tenn "alkynyl" includes di- and tri-ynes;
- the "cycloalkyl", or "alicyclic" group used either alone or in combination with other radicals, is selected from a cyclic radical containing three to six carbons, more preferably cyclopropyl, cyclobutyl, cyclopentyl, cyclohexyl and the like; The terms "bicycloalkyl" means more than one cycloalkyl groups fused together;
- the "cycloalkenyl" group used either alone or in combination with other radicals, are preferably selected from cyclopropenyl, 1-cyclobutenyl, 2-cylobutenyl, 1-cyclopentenyl, 2-cyclopentenyl, 3-cyclopentenyl, 1-cyclohexenyl, 2-cyclohexenyl, 3-cyclohexenyl and the like; The terms "bicycloalkenyl" means more than one cycloalkenyl groups fused together;
- the "alkoxy" group used either alone or in combination with other radicals, is selected from groups containing an alkyl radical, as defined above, attached directly to an oxygen atom, more preferably groups selected from methoxy, ethoxy, n-propoxy, iso-propoxy, n-butoxy, t-butoxy, iso-butoxy, pentyloxy, hexyloxy, and the like;
- the "cycloalkoxy" group used either alone or in combination with other radicals, is is selected from a cyclic radical containing three to seven carbons, more preferably cyclopropyloxy, cyclobutylxoy, cyclopentyloxy, cyclohexyloxy and the like; The terms "bicycloalkyloxy" means more than one cycloalkyl groups fused together;
- the "alkenoxy" group used either alone or in combination with other radicals, is selected from groups containing an alkenyl radical, as defined above, attached to an oxygen atom, more preferably selected from vinyloxy, allyloxy, butenoxy, pentenoxy, hexenoxy, and the like;
- the "haloalkyl" group is selected from an alkyl radical, as defined above, suitably substituted with one or more halogens; such as perhaloalkyl, more preferably, perfluoro (C₁-C₆)alkyl such as fluoromethyl, difluoromethyl, trifluoromethyl, fluoroethyl, difluoroethyl, trifluoroethyl, mono or polyhalo substituted methyl, ethyl, propyl, butyl, pentyl or hexyl groups;
- the "haloalkoxy" group is selected from suitable haloalkyl, as defined above, directly attached to an oxygen atom, more preferably groups selected from fluoromethoxy, chloromethoxy, fluoroethoxy, chloroethoxy and the like;
- the "perhaloalkoxy" group is selected from a suitable perhaloalkyl radical, as defined above, directly attached to an oxygen atom, more preferably groups selected from trifluoromethoxy, trifluoroethoxy, and the like;
- the "aryl" or "aromatic" group used either alone or in combination with other radicals, is selected from a suitable aromatic system containing one, two or three rings wherein such rings may be attached together in a pendant manner or may be fused, more preferably the groups are selected from phenyl, naphthyl, tetrahydronaphthyl, indane, biphenyl, and the like;
- the "aryloxy" group used either alone or in combination with other radicals, is selected from groups containing an aryl radical, as defined above, attached directly to an oxygen atom, more preferably groups selected from phenoxy, naphthyloxy, tetrahydronaphthyloxy, biphenyloxy, and the like;
- ;
- the groups "heteroaryloxy", "heteroaralkoxy", "heterocycloxy", "heterocylylalkoxy" are selected from suitable heteroaryl, heteroarylalkyl, heterocyclyl, heterocylylalkyl groups respectively, as defined above, attached to an oxygen atom;
- the "acyl" group used either alone or in combination with other radicals, is selected from a radical containing one to eight carbons, more preferably selected from formyl, acetyl, propanoyl, butanoyl, iso-butanoyl, pentanoyl, hexanoyl, heptanoyl, benzoyl and the like, which may be substituted;
- the "acyloxy" group used either alone or in combination with other radicals, is selected from a suitable acyl group, as defined above, directly attached to an oxygen atom, more preferably such groups are selected from acetyloxy, propionyloxy, butanoyloxy, iso-butanoyloxy, benzoyloxy and the like;
- the "acylamino" group used either alone or in combination with other radicals, is selected from a suitable acyl group as defined earlier, attached to an amino radical, more preferably such groups are selected from CH₃CONH, C₂H₅CONH, C₃H₇CONH, C₄H₉CONH, C₆H₅CONH and the like, which may be substituted;
- the "mono-substituted amino" group used either alone or in combination with other radicals, represents an amino group substituted with one group selected from (C₁-C₆)alkyl, substituted alkyl, aryl, substituted aryl or arylalkyl groups as defined earlier, more preferably such groups are selected from methylamine, ethylamine, n-propylamine, n-butylamine, n-pentylamine and the like;
- the 'disubstituted amino" group used either alone or in combination with other radicals, represents an amino group, substituted with two radicals that may be same or different selected from (C₁-C₆)alkyl, substituted alkyl, aryl, substituted aryl, or arylalkyl groups, as defined above, more preferably the groups are selected from dimethylamino, methylethylamino, diethylamino, phenylmethyl amino and the like;
- the "arylamino" used either alone or in combination with other radicals, represents an aryl group, as defined above, linked through amino having a free valence bond from the nitrogen atom, more preferably the groups are selected from phenylamino, naphthylamino, N-methyl anilino and the like;
- the "oxo" or "carbonyl" group used either alone (-C=O-) or in combination with other radicals such as alkyl described above, for e.g. "alkylcarbonyl", denotes a carbonyl radical (-C=O-) substituted with an alkyl radical described above such as acyl or alkanoyl;
- the "carboxylic acid" group, used alone or in combination with other radicals, denotes a -COOH group, and includes derivatives of carboxylic acid such as esters and amides;
- the "ester" group used alone or in combination with other radicals, denotes -COO-group, and includes carboxylic acid derivatives, more preferably the ester moieties are selected from alkoxycarbonyl, such as methoxycarbonyl, ethoxycarbonyl, and the like, which may optionally be substituted; aryloxycarbonyl group such as phenoxycarbonyl, napthyloxycarbonyl, and the like, which may optionally be substituted; aralkoxycarbonyl group such as benzyloxycarbonyl, phenethyloxycarbonyl, napthylmethoxycarbonyl, and the like, which may optionally be substituted; heteroaryloxycarbonyl, heteroaralkoxycarbonyl, wherein the heteroaryl group, is as defined above, which may optionally be substituted; heterocyclyloxycarbonyl, where the heterocyclic group, as defined earlier, which may optionally be substituted;
- the "amide" group used alone or in combination with other radicals, represents an aminocarbonyl radical (H₂N-C=O), wherein the amino group is mono- or di-substituted or unsubstituted, more preferably the groups are selected from methyl amide, dimethyl amide, ethyl amide, diethyl amide, and the like;
- the "aminocarbonyl" group used either alone or in combination with other radicals, may be selected from "aminocarbonyl', 'aminocarbonylalkyl", "n-alkylaminocarbonyl", "N-arylaminocarbonyl", "N,N-dialkylaminocarbonyl", "N-alkyl-N-arylaminocarbonyl", "N-alkyl-N-hydroxyaminocarbonyl", and "N-alkyl-N-hydroxyaminocarbonylalkyl", each of them being optionally substituted. The terms "N-alkylaminocabonyl" and "N,N-dialkylaminocarbonyl" denotes aminocarbonyl radicals, as defined above, which have been substituted with one alkyl radical and with two alkyl radicals, respectively. Preferred are "lower alkylaminocarbonyl" having lower alkyl radicals as described above attached to aminocarbonyl radical. The terms "N-arylaminocarbonyl" and "N-alkyl-N-arylaminocarbonyl" denote amiocarbonyl radicals substituted, respectively, with one aryl radical, or one alkyl, and one aryl radical. The term "aminocarbonylalkyl" includes alkyl radicals substituted with aminocarbonyl radicals;
- the "hydroxyalkyl" group used either alone or in combination with other radicals, is selected from an alkyl group, as defined above, substituted with one or more hydroxy radicals, more preferably the groups are selected from hydroxymethyl, hydroxyethyl, hydroxypropyl, hydroxybutyl, hydroxypentyl, hydroxyhexyl and the like;
- the "aminoalkyl" group used alone or in combination with other radicals, denotes an amino (-NH₂) moiety attached to an alkyl radical, as defined above, which may be substituted, such as mono- and di-substituted aminoalkyl. The term "alkylamino" used herein, alone or in combination with other radicals, denotes an alkyl radical, as defined above, attached to an amino group, which may be substituted, such as mono- and di-substituted alkylamino;
- the "alkoxyalkyl" group used alone or in combination with other radicals, denotes an alkoxy group, as defined above, attached to an alkyl group as defined above, more preferably the groups may be selected from methoxymethyl, ethoxymethyl, methoxyethyl, ethoxyethyl and the like;
- the "alkylthio" group used either alone or in combination with other radicals, denotes a straight or branched or cyclic monovalent substituent comprising an alkyl group as defined above, linked through a divalent sulfur atom having a free valence bond from the sulfur atom, more preferably the groups may be selected from methylthio, ethylthio, propylthio, butylthio, pentylthio and the like or cyclic alkylthio selected from cyclopropylthio, cyclobutylthio, cyclopentylthio, cyclohexylthio and the like, which may be optionally substituted;
- the "thioalkyl" group used either alone or in combination with other radicals, denotes an alkyl group, as defined above, attached to a group of formula -SR', where R' represents hydrogen, alkyl or aryl group, e.g. thiomethyl, methylthiomethyl, phenylthiomethyl and the like, which may be optionally substituted.
- the "arylthio" group used either alone or in combination with other radicals, denotes a comprising an aryl group as defined above, linked through a divalent sulfur atom having a free valence bond from the sulfur atom, more preferably the groups may be selected from phenylthio, naphthylthio, tetrahydronaphthylthio, indanethio, biphenylthio, and the like;
- the "heterocyclylthio" group used either alone or in combination with other radicals, denotes a comprising an heterocyclyl group as defined above, linked through a divalent sulfur atom having a free valence bond from the sulfur atom, more preferably the groups may be selected from aziridinylthio, azetidinylthio, pyrrolidinylthio, imidazolidinylthio, piperidinylthio, piperazinylthio, 2-oxopiperidinylthio, 4-oxopiperidinylthio, 2-oxopiperazinylthio, 3-oxopiperazinylthio, morpholinylthio, thiomorpholinylthio, 2-oxomorpholinylthio, azepinylthio, diazepinylthio, oxapinylthio, thiazepinylthio, oxazolidinylthio, thiazolidinylthio, dihydrothiophenethio, dihydropyranthio, dihydrofuranthio, dihydrothiazolethio, benzopyranylthio, benzopyranonylthio, benzodihydrofuranylthio, benzodihydrothienylthio, pyrazolopyrimidonylthio, azaquinazolinoylthio, thienopyrimidonylthio, quinazolonylthio, pyrimidonylthio, benzoxazinylthio, benzoxazinonylthio, benzothiazinylthio, benzothiazinonylthio, thieno piperidinylthio, and the like;
- the "heteroarylthio" group used either alone or in combination with other radicals, denotes a comprising an heteroaryl group as defined above, linked through a divalent sulfur atom having a free valence bond from the sulfur atom, more preferably the groups may be selected from pyridylthio, thienylthio, furylthio, pyrrolylthio, oxazolylthio, thiazolylthio, isothiazolylthio, imidazolylthio, isoxazolylthio, oxadiazolylthio, thiadiazolylthio, triazolylthio, tetrazolylthio, benzofuranylthio, benzothienylthio, indolinylthio, indolylthio, azaindolylthio, azaindolinylthio, pyrazolopyrimidinylthio, azaquinazolinylthio, pyridofuranylthio, pyridothienylthio, thienopyrimidylthio, quinolinylthio, pyrimidinylthio, pyrazolylthio, quinazolinylthio, pyridazinylthio, triazinylthio, benzimidazolylthio, benzotriazolylthio, phthalazynilthio, naphthylidinylthio, purinylthio, carbazolylthio, phenothiazinylthio, phenoxazinylthio, benzoxazolylthio, benzothiazolylthio and the like;
- the "alkoxycarbonylamino" group used alone or in combination with other radicals, is selected from a suitable alkoxycarbonyl group, as defined above, attached to an amino group, more preferably methoxycarbonylamino, ethoxycarbonylamino, and the like;
- the "aminocarbonylamino", "alkylaminocarbonylamino", "dialkylaminocarbonylamino" groups used alone or in combination with other radicals, is a carbonylamino (-CONH₂) group, attached to amino(NH₂), alkylamino group or dialkylamino group respectively, where alkyl group is as defined above;
- the "amidino" group used either alone or in combination with other radicals, represents a -C(=NH)-NH₂ radical; the "alkylamidino" group represents an alkyl radical, as described above, attached to an amidino group;
- the "alkoxyamino" group used either alone or in combination with other radicals, represents a suitable alkoxy group as defined above, attached to an amino group;
- the "hydroxyamino" group used either alone or in combination with other radicals, represents a -NHOH moiety, and may be optionally substituted with suitable groups selected from those described above;
- the "sulfinyl" group or "sulfinyl derivatives" used alone or in combination with other radicals, represents a bivalent group, -SO- or RₓSO, where Rₓ is an optionally substituted alkyl, aryl, heteroaryl, heterocyclyl, group selected from those described above;
- the "sulfonyl" group or "sulfones derivatives" used either alone or in combination with other radicals, with other terms such as alkylsulfonyl, represents a divalent radical -SO₂-, or RₓSO₂-, where Rₓ is as defined above. More preferably, the groups may be selected from "alkylsulfonyl" wherein suitable alkyl radicals, selected from those defined above, is attached to a sulfonyl radical, such as methylsulfonyl, ethylsulfonyl, propylsulfonyl and the like, "arylsulfonyl" wherein an aryl radical, as defined above, is attached to a sulfonyl radical, such as phenylsulfonyl and the like;
- the "sulfanyl" group or "sulfanyl derivatives" used alone or in combination with other radicals, represents a bivalent group, -S- or RxS, where Rx is an optionally substituted alkyl, aryl, heteroaryl, heterocyclyl, group selected from those described above;
- the "sulfonyloxy" group used either alone or in combination with other radicals, with other terms such as alkylsulfonyloxy, represents a divalent radical -SO₂-O-, or RₓSO₂-O-, where Rₓ is as defined above. More preferably, the groups may be selected from "alkylsulfonyloxy" wherein suitable alkyl radicals, selected from those defined above, is attached to a sulfonyl radical, such as methylsulfonyl, ethylsulfonyl, propylsulfonyl and the like, "arylsulfonyloxy" wherein an aryl radical, as defined above, is attached to a sulfonyloxy radical, such as phenylsulfonyloxy and the like.

Suitable groups and substituents on the groups may be selected from those described anywhere in the specification.

Particularly useful compounds may be selected from
N-(benzyloxy)-2-methyl-5-((5-methyl-2-phenyloxazol-4-yl)methyl)-1,3-dioxane-2-carboxamide;
N-(benzyloxy)-2-methyl-5-c-((5-methyl-2-phenyloxazol-4-yl)methyl)-1,3-dioxane-2-carboxamide;
N-(benzyloxy)-2-methyl-5-t-((5-methyl-2-phenyloxazol-4-yl)methyl)-1,3-dioxane-2-carboxamide;
N-hydroxy-2-methyl-5-((5-methyl-2-phenyloxazol-4-yl)methyl)-1,3-dioxane-2-carboxamide;
5-c-(3-(9H-carbazol-9-yl)propyl)-N-hydroxy-2-methyl-1,3-dioxane-2-carboxamide; N-hydroxy-2-methyl-5-c-((5-methyl-2-phenyloxazol-4-yl)methyl)-1,3-dioxane-2-carboxam ide;
N-hydroxy-2-methyl-5-t-((5-methyl-2-phenyloxazol-4-yl)methyl)-1,3-dioxane-2-carboxamide;
2-methyl-5-((5-methyl-2-phenyloxazol-4-yl)methyl)-N-((tetrahydro-2H-pyran-4-yl)methyl)-1,3-dioxane-2-carboxamide;
2-methyl-5-c-((5-methyl-2-phenyloxazol-4-yl)methyl)-N-((tetrahydro-2H-pyran-4-yl)methyl)-1,3-dioxane-2-carboxamide;
2-methyl-5-t-((5-methyl-2-phenyloxazol-4-yl)methyl)-N-((tetrahydro-2H-pyran-4-yl)methyl)-1,3-dioxane-2-carboxamide;
(2-methyl-5-((5-methyl-2-phenyloxazol-4-yl)methyl)-1,3-dioxan-2-yl)(morpholino)methanone;
(2-methyl-5-*c*-((5-methyl-2-phenyloxazol-4-yl)methyl)-1,3-dioxan-2-yl)(morpholino)methanone;
(2-methyl-5-*t*-((5-methyl-2-phenyloxazol-4-yl)methyl)-1,3-dioxan-2-yl)(morpholino)methanone;
(2-methyl-5-*c*-((5-methyl-2-phenyloxazol-4-yl)methyl)-1,3-dioxan-2-yl)(4-methylpiperazin-1-yl)methanone;
(2-methy)-5-*c*-((5-methyl-2-phenyloxazol-4-yl)methyl)-1,3-dioxan-2-yl)(piperidin-1-yl)methanone;
(2-methyl-5-*c*-((5-methyl-2-phenyloxazol-4-yl)methyl)-1,3-dioxan-2-yl)(thiomorpholino)methanone;
(1,1-dioxidothiomorpholino)(2-methyl-5-*c*-((5-methyl-2-phenyloxazol-4-yl)methyl)-1,3-dioxan-2-yl)methanone;
2-methyl-5-((5-methyl-2-phenyloxazol-4-yl)methyl)-N-(2-morpholino-2-oxoethyl)-1,3-dioxane-2-carboxam ide;
2-methyl-5-*c*-((5-methyl-2-phenyloxazol-4-yl)methyl)-N-(2-morpholino-2-oxoethyl)-1,3-dioxane-2-carboxamide;
2-methyl-5-*t*-((5-methyl-2-phenyloxazol-4-yl)methyl)-N-(2-morpholino-2-oxoethyl)-1,3-dioxane-2-carboxamide;
2-methyl-5-*c*-((5-methyl-2-phenyloxazol-4-yl)methyl)-N-(2-(4-methylpiperazin-1-yl)-2-oxoethyl)-1,3-dioxane-2-carboxamide;
2-methyl-5-*c*-((5-methyl-2-phenyloxazol-4-yl)methyl)-N-(2-oxo-2-(piperidin-1-yl)ethyl)-1,3-dioxane-2-carboxamide;
2-methyl-5-*c*-((5-methyl-2-phenyloxazol-4-yl)methyl)-N-(2-oxo-2-thiomorpholinoethyl)-1,3-dioxane-2-carboxamide;
N-(2-(1,1-dioxidothiomorpholino)-2-oxoethyl)-2-methyl-5-*c*-((5-methyl-2-phenyloxazol-4-yl)methyl)-1,3-dioxane-2-carboxamide;
2-methyl-5-*c*-((5-methyl-2-phenyloxazol-4-yl)methyl)-N-(2-oxo-2-(((tetrahydro-2H-pyran-4-yl)methyl)amino)ethyl)-1,3-dioxane-2-carboxamide;
2-methyl-5-*c*-((5-methyl-2-phenyloxazol-4-yl)methyl)-N-(3-morpholino-3-oxopropyl)-1,3-dioxane-2-carboxamide;
2-methyl-5-*c*-((5-methyl-2-phenyloxazol-4-yl)methyl)-N-(3-oxo-3-(((tetrahydro-2H-pyran-4-yl)methyl)amino)propyl)-1,3-dioxane-2-carboxamide;
2-methyl-5-*c*-((5-methyl-2-phenyloxazol-4-yl)methyl)-N-(3-oxo-3-thiomorpholinopropyl)-1,3-dioxane-2-carboxamide;
N-(3-(1,1-dioxidothiomorpholino)-3-oxopropyl)-2-methyl-5-*c*-((5-methyl-2-phenyloxazol-4-yl)methyl)-1,3-dioxane-2-carboxamide;
2-methyl-5-*c*-((5-methyl-2-phenyloxazol-4-yl)methyl)-N-(3-oxo-3-(piperidin-1-yl)propyl)-1,3-dioxane-2-carboxamide;
2-methyl-5-*c*-((5-methyl-2-phenyloxazol-4-yl)methyl)-N-(3-(4-methy)piperazin-1-yl)-3-oxopropyl)-1,3-dioxane-2-carboxamide;
2-methyl-5-((5-methyl-2-phenyloxazol-4-yl)methyl)-N-(3-oxo-1-phenylbutan-2-yl)-1,3-dioxane-2-carboxamide;
N-benzyloxy-2-methyl-5-(2-(5-methyl-2-phenyloxazol-4-yl)ethyl)-1,3-dioxane-2-carboxamide;
N-hydroxy-2-methyl-5-(2-(5-methyl-2-phenyloxazol-4-yl)ethyl)-1,3-dioxane-2-carboxamide;
N-hydroxy-2-methyl-5-*c*-(2-(5-methyl-2-phenyloxazol-4-yl)ethyl)-1,3-dioxane-2-carboxamide;
N-hydroxy-2-methyl-5-*t*-(2-(5-methyl-2-phenyloxazol-4-yl)ethyl)-1,3-dioxane-2-carboxam ide;
2-methyl-5-(2-(5-methyl-2-phenyloxazol-4-yl)ethyl)-N-((tetrahydro-2H-pyran-4-yl)methyl)-1,3-dioxane-2-carboxamide;
2-methyl-5-*c*-(2-(5-methyl-2-phenyloxazol-4-yl)ethyl)-N-((tetrahydro-2H-pyran-4-yl)methyl)-1,3-dioxane-2-carboxamide;
2-methyl-5-*t*-(2-(5-methyl-2-phenyloxazol-4-yl)ethyl)-N-((tetrahydro-2H-pyran-4-yl)methyl)-1,3-dioxane-2-carboxamide;
(2-methyl-5-(2-(5-methyl-2-phenyloxazol-4-yl)ethyl)-1,3-dioxan-2-yl)(morpholino)methanone;
(2-methyl-5-*c*-(2-(5-methyl-2-phenyloxazol-4-yl)ethyl)-1,3-dioxan-2-yl)(morpholino)methanone;
(2-methyl-5-*t*-(2-(5-methyl-2-phenyloxazol-4-yl)ethyl)-1,3-dioxan-2-yl)(morpholino)methanone;
(2-methyl-5-*c*-(2-(5-methyl-2-phenyloxazol-4-yl)ethyl)-1,3-dioxan-2-yl)(4-methylpiperazin-1-yl)methanone;
(2-methyl-5-*c*-(2-(5-methyl-2-phenyloxazol-4-yl)ethyl)-1,3-dioxan-2-yl)(piperidin-1-yl)methanone;
(2-methyl-5-*c*-(2-(5-methyl-2-phenyloxazol-4-yl)ethyl)-1,3-dioxan-2-yl)(thiomorpholino)methanone;
(1,1-dioxidothiomorpholino)(2-methyl-5-*c*-(2-(5-methyl-2-phenyloxazol-4-yl)ethyl)-1,3-dioxan-2-yl)methanone;
2-methyl-5-(2-(5-methyl-2-phenyloxazol-4-yl)ethyl)-N-(2-morpholino-2-oxoethyl)-1,3-dioxane-2-carboxamide;
2-methyl-5-*c*-(2-(5-methyl-2-phenyloxazol-4-yl)ethyl)-N-(2-morpholino-2-oxoethyl)-1,3-dioxane-2-carboxamide;
2-methyl-5-*t*-(2-(5-methyl-2-phenyloxazol-4-yl)ethyl)-N-(2-morpholino-2-oxoethyl)-1,3-dioxane-2-carboxamide;
2-methyl-5-*c*-(2-(5-methyl-2-phenyloxazol-4-yl)ethyl)-N-(2-oxo-2-(((tetrahydro-2H-pyran-4-yl)methyl)amino)ethyl)-1,3-dioxane-2-carboxamide;
2-methyl-5-*c*-(2-(5-methyl-2-phenyloxazol-4-yl)ethyl)-N-(2-(4-methylpiperazin-1-yl)-2-oxoethyl)-1,3-dioxane-2-carboxamide;
2-methyl-5-*c*-(2-(5-methyl-2-phenyloxazol-4-yl)ethyl)-N-(2-oxo-2-thiomorpholinoethyl)-1,3-dioxane-2-carboxamide;
2-methyl-5-*c*-(2-(5-methyl-2-phenyloxazol-4-yl)ethyl)-N-(2-oxo-2-(piperidin-1-yl)ethyl)-1,3-dioxane-2-carboxamide;
2-methyl-5-(2-(5-methyl-2-phenyloxazol-4-yl)ethyl)-N-(3-oxo-1-phenylbutan-2-yl)-1,3-dioxane-2-carboxamide;
2-methyl-5-(2-(5-methyl-2-phenyloxazol-4-yl)ethyl)-N-(2-oxo-2-((3-oxo-1-phenylbutan-2-yl)amino)ethyl)-1,3-dioxane-2-carboxamide;
N-benzyloxy-2-methyl-5-((5-methyl-2-(p-tolyl)oxazol-4-yl)methyl)-1,3-dioxane-2-carboxamide;
N-benzyloxy-2-methyl-5-*c*-((5-methyl-2-(p-tolyl)oxazol-4-yl)methyl)-1,3-dioxane-2-carboxam ide;
N-benzyloxy-2-methyl-5-*t*-((5-methyl-2-(p-tolyl)oxazol-4-yl)methyl)-1,3-dioxane-2-carboxam ide;
N-hydroxy-2-methyl-5-((5-methyl-2-(p-tolyl)oxazol-4-yl)methyl)-1,3-dioxane-2-carboxamide;
N-hydroxy-2-methyl-5-*c*-((5-methyl-2-(p-tolyl)oxazol-4-yl)methyl)-1,3-dioxane-2-carboxamide;
N-hydroxy-2-methyl-5-*t*-((5-methyl-2-(p-tolyl)oxazol-4-yl)methyl)-1,3-dioxane-2-carboxamide;
2-methyl-5-((5-methyl-2-(p-tolyl)oxazol-4-yl)methyl)-N-((tetrahydro-2H-pyran-4-yl)methyl)-1,3-dioxane-2-carboxamide;
2-methyl-5-*c*-((5-methyl-2-(p-tolyl)oxazol-4-yl)methyl)-N-((tetrahydro-2H-pyran-4-yl)methyl)-1,3-dioxane-2-carboxamide;
2-methyl-5-*t*-((5-methyl-2-(p-tolyl)oxazol-4-yl)methyl)-N-((tetrahydro-2H-pyran-4-yl)methyl)-1,3-dioxane-2-carboxamide;
(2-methyl-5-((5-methyl-2-(p-tolyl)oxazol-4-yl)methyl)-1,3-dioxan-2-yl)(morpholino)methanone;
(2-methyl-5-*c*-((5-methyl-2-(p-tolyl)oxazol-4-yl)methyl)-1,3-dioxan-2-yl)(morpholino)methanone;
(2-methyl-5-*t*-((5-methyl-2-(p-tolyl)oxazol-4-yl)methyl)-1,3-dioxan-2-yl)(morpholino)methanone;
2-methyl-5-((5-methyl-2-(p-tolyl)oxazol-4-yl)methyl)-N-(2-morpholino-2-oxoethyl)-1,3-dioxane-2-carboxamide;
2-methyl-5-*c*-((5-methyl-2-(p-tolyl)oxazol-4-yl)methyl)-N-(2-morpholino-2-oxoethyl)-1,3-dioxane-2-carboxamide;
2-methyl-5-*t*-((5-methyl-2-(p-tolyl)oxazol-4-yl)methyl)-N-(2-morpholino-2-oxoethyl)-1,3-dioxane-2-carboxamide;
N-(benzyloxy)-5-((2-(tert-butyl)-5-methyloxazol-4-yl)methyl)-2-methyl-1,3-dioxane-2-carboxamide;
5-((2-(tert-butyl)-5-methyloxazol-4-yl)methyl)-N-hydroxy-2-methyl-1,3-dioxane-2-carboxamide;
(5-((2-(tert-butyl)-5-methyloxazol-4-yl)methyl)-2-methyl-1,3-dioxan-2-yl)(morpholino)methanone;
2-methyl-5-*c*-((5-methyl-2-(5-methylthiophen-2-yl)oxazol-4-yl)methyl)-N-((tetrahydro-2H-pyran-4-yl)methyl)-1,3-dioxane-2-carboxamide;
2-methyl-5-*t*-((5-methyl-2-(5-methylthiophen-2-yl)oxazol-4-yl)methyl)-N-((tetrahydro-2H-pyran-4-yl)methyl)-1,3-dioxane-2-carboxamide;
2-methyl-5-*c*-((5-methyl-2-(5-methylthiophen-2-yl)oxazol-4-yl)methyl)-1,3-dioxan-2-yl)(morpholino)methanone;
2-methyl-5-*t*-((5-methyl-2-(5-methylthiophen-2-yl)oxazol-4-yl)methyl)-1,3-dioxan-2-yl)(morpholino)methanone;
N-benzyloxy-2-methyl-5-*c*-((5-methyl-2-(pyridin-4-yl)oxazol-4-yl)methyl)-1,3-dioxane-2-carboxamide;
N-benzyloxy-2-methyl-5-*t*-((5-methyl-2-(pyridin-4-yl)oxazol-4-yl)methyl)-1,3-dioxane-2-carboxamide;
N-hydroxy-2-methyl-5-*c*-((5-methyl-2-(pyridin-4-yl)oxazol-4-yl)methyl)-1,3-dioxane-2-carboxamide;
N-hydroxy-2-methyl-5-*t*-((5-methyl-2-(pyridin-4-yl)oxazol-4-yl)methyl)-1,3-dioxane-2-carboxamide;
(2-methyl-5-*c*-((5-methyl-2-(pyridin-4-yl)oxazol-4-yl)methyl)-1,3-dioxan-2-yl)(morpholino)methanone;
(2-methyl-5-*t*-((5-methyl-2-(pyridin-4-yl)oxazol-4-yl)methyl)-1,3-dioxan-2-yl)(morpholino)methanone;
2-methyl-5-*c*-((5-methyl-2-(pyridin-4-yl)oxazol-4-yl)methyl)-N-((tetrahydro-2H-pyran-4-yl)methyl)-1,3-dioxane-2-carboxamide;
2-methyl-5-*t*-((5-methyl-2-(pyridin-4-yl)oxazol-4-yl)methyl)-N-((tetrahydro-2H-pyran-4-yl)methyl)-1,3-dioxane-2-carboxamide;
2-methyl-5-*c*-((5-methyl-2-(pyridin-4-yl)oxazol-4-yl)methyl)-N-(2-morpholino-2-oxoethyl)-1,3-dioxane-2-carboxamide;
2-methyl-5-*t*-((5-methyl-2-(pyridin-4-yl)oxazol-4-yl)methyl)-N-(2-morpholino-2-oxoethyl)-1,3-dioxane-2-carboxamide;
2-methyl-5-*c*-((5-methyl-2-(pyridin-4-yl)oxazol-4-yl)methyl)-N-(2-oxo-2-(((tetrahydro-2H-pyran-4-yl)methyl)amino)ethyl)-1,3-dioxane-2-carboxamide;
2-methyl-5-*t*-((5-methyl-2-(pyridin-4-yl)oxazol-4-yl)methyl)-N-(2-oxo-2-(((tetrahydro-2H-pyran-4-yl)methyl)amino)ethyl)-1,3-dioxane-2-carboxamide;
N-benzyloxy-2-methyl-5-((4-methyl-2-(4-(trifluoromethyl)phenyl)thiazol-5-yl)methyl)-1,3-dioxane-2-carboxamide;
N-hydroxy-2-methyl-5-((4-methyl-2-(4-(trifluoromethyl)phenyl)thiazol-5-yl)methyl)-1,3-dioxane-2-carboxamide;
1-(2-methyl-5-((4-methyl-2-(4-(trifluoromethyl)phenyl)thiazol-5-yl)methyl)-1,3-dioxan-2-yl)-N-((tetrahydro-2H-pyran-4-yl)methyl)methanamine;
(2-methyl-5-((4-methyl-2-(4-(trifluoromethyl)phenyl)thiazol-5-yl)methyl)-1,3-dioxan-2-yl)(morpholino)methanone;
(2-methyl-5-*c*-((4-methyl-2-(4-(trifluoromethyl)phenyl)thiazol-5-yl)methyl)-1,3-dioxan-2-yl)(morpholino)methanone;
(2-methyl-5-t-((4-methyl-2-(4-(trifluoromethyl)phenyl)thiazol-5-yl)methyl)-1,3-dioxan-2-yl)(morpholino)methanone;
5-*c*-(3-(9H-carbazol-9-yl)propyl)-N-hydroxy-2-methyl-1,3-dioxane-2-carboxamide; N-hydroxy-5-c-(3-(10H-phenothiazin-10-yl)propyl)-2-methyl-1,3-dioxane-2-carboxamide;
N-(benzyloxy)-5-((3-(tert-butyl)-1-(p-tolyl)-1H-pyrazol-5-yl)methyl)-2-methyl-1,3-dioxane-2-carboxamide;
N-(hydroxy)-5-*c*-((3-(tert-butyl)-1-(p-tolyl)-1H-pyrazol-5-yl)methyl)-2-methyl-1,3-dioxane-2-carboxamide;
N-(hydroxy)-5-*t*-((3-(tert-butyl)-1-(p-tolyl)-1H-pyrazol-5-yl)methyl)-2-methyl-1,3-dioxane-2-carboxam ide;
5-((3-(tert-butyl)-1-(p-tolyl)-1H-pyrazol-5-yl)methyl)-2-methyl-N-((tetrahydro-2H-pyran-4-yl)methyl)-1,3-dioxane-2-carboxamide;
5-((3-(tert-butyl)-1-(p-tolyl)-1H-pyrazol-5-yl)methyl)-2-methyl-N-(2-oxo-2-(((tetrahydro-2H-pyran-4-yl)methyl)amino)ethyl)-1,3-dioxane-2-carboxamide;
(5-((3-(tert-butyl)-1-(p-tolyl)-1H-pyrazol-5-yl)methyl)-2-methyl-1,3-dioxan-2-yl)(morpholino)methanone;
5-((3-(tert-butyl)-1-(p-tolyl)-1H-pyrazol-5-yl)methyl)-2-methyl-N-(2-morpholino-2-oxoethyl)-1,3-dioxane-2-carboxamide;
(5-((3-(tert-butyl)-1-(p-tolyl)-1H-pyrazol-5-yl)methyl)-2-methyl-1,3-dioxan-2-yl)(4-methylpiperazin-1-yl)methanone;
N-(benzyloxy)-5-*c*-((2-(4-methoxyphenyl)-5-methyloxazol-4-yl)methyl)-2-methyl-1,3-dioxane-2-carboxamide;
N-(benzyloxy)-5-*t*-((2-(4-methoxyphenyl)-5-methyloxazol-4-yl)methyl)-2-methyl-1,3-dioxane-2-carboxamide;
N-hydroxy-5-*c*-((2-(4-methoxyphenyl)-5-methyloxazol-4-yl)methyl)-2-methyl-1,3-dioxane-2-carboxamide;
N-hydroxy-5-*t*-((2-(4-methoxyphenyl)-5-methyloxazol-4-yl)methyl)-2-methyl-1,3-dioxane-2-carboxamide;
(5-*c*-((2-(4-methoxyphenyl)-5-methyloxazol-4-yl)methyl)-2-methyl-1,3-dioxan-2-yl)(morpholino)methanone;
(5-*t*-((2-(4-methoxyphenyl)-5-methyloxazol-4-yl)methyl)-2-methyl-1,3-dioxan-2-yl)(morpholino)methanone;
5-*c*-((2-(4-methoxyphenyl)-5-methyloxazol-4-yl)methyl)-2-methyl-N-((tetrahydro-2H-pyran-4-yl)methyl)-1,3-dioxane-2-carboxamide;
5-*t*-((2-(4-methoxyphenyl)-5-methyloxazol-4-yl)methyl)-2-methyl-N-((tetrahydro-2H-pyran-4-yl)methyl)-1,3-dioxane-2-carboxamide;
(5-*c*-((2-(4-methoxyphenyl)-5-methyloxazol-4-yl)methyl)-2-methyl-1,3-dioxan-2-yl)(4-methylpiperazin-1-yl)methanone;
(5-*t*-((2-(4-methoxyphenyl)-5-methyloxazol-4-yl)methyl)-2-methyl-1,3-dioxan-2-yl)(4-methylpiperazin-1-yl)methanone;
N-(benzyloxy)-2-methyl-5-*c*-((5-methyl-2-(4-(trifluoromethyl)phenyl)oxazol-4-yl)methyl)-1,3-dioxane-2-carboxamide;
N-(benzyloxy)-2-methyl-5-*t*-((5-methyl-2-(4-(trifluoromethyl)phenyl)oxazol-4-yl)methyl)-1,3-dioxane-2-carboxamide;
N-hydroxy-2-methyl-5-*c*-((5-methyl-2-(4-(trifluoromethyl)phenyl)oxazol-4-yl)methyl)-1,3-dioxane-2-carboxamide;
N-hydroxy-2-methyl-5-*t*-((5-methyl-2-(4-(trifluoromethyl)phenyl)oxazol-4-yl)methyl)-1,3-dioxane-2-carboxamide;
(2-methyl-5-*c*-((5-methyl-2-(4-(trifluoromethyl)phenyl)oxazol-4-yl)methyl)-1,3-dioxan-2-yl)(morphol ino)methanone;
(2-methyl-5-*t*-((5-methyl-2-(4-(trifluoromethyl)phenyl)oxazol-4-yl)methyl)-1,3-dioxan-2-yl)(morpholino)methanone;
2-methyl-5-*c*-((5-methyl-2-(4-(trifluoromethyl)phenyl)oxazol-4-yl)methyl)-N-((tetrahydro-2H-pyran-4-yl)methyl)-1,3-dioxane-2-carboxamide;
2-methyl-5-*t*-((5-methyl-2-(4-(trifluoromethyl)phenyl)oxazol-4-yl)methyl)-N-((tetrahydro-2H-pyran-4-yl)methyl)-1,3-dioxane-2-carboxamide;

The novel compounds of this invention may be prepared using the reactions and techniques as shown in scheme below and described in this section. The reactions are performed in solvents appropriate to the reagents and materials employed and are suitable for the transformations being effected. It is understood by those skilled in the art that the nature and order of the synthetic steps presented may be varied for the purpose of optimizing the formation of the compounds of the present invention. It will also be well appreciated that one or more of the reactants may be protected and deprotected for facile synthesis by techniques known to persons skilled in the art. It will also be appreciated that one or more of the compounds of the present invention may exist in stereoisomeric and/or diastereomeric forms. Such stereoisomers and/or diastereoisomers as well as their optical antipodes are to be construed to be within the scope of the present invention. It will also be well appreciated that one or more of these compounds may be converted to their salts and other derivatives based on the specific groups present on the compounds, which can be well comprehended by persons skilled in the art. Such salts and/or other derivatives, as the case may be should also be construed to be within the scope of the present invention.

**Scheme 1**: The compounds of general formula (**I**) wherein all the symbols are as defined earlier, may be prepared by reactions outlined in **Scheme 1** below which comprises

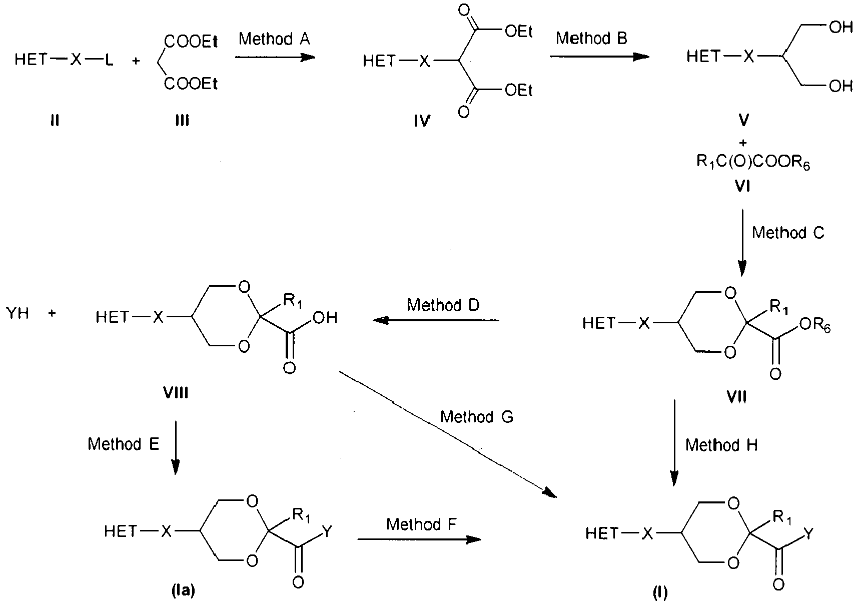

**Method A:** Compounds of formula (**IV**) wherein 'HET' and 'X' are as defined earlier may be prepared by reacting compounds of general formula (**II**) where 'L' is a suitable leaving group and 'HET' and 'X' are as defined earlier with diethyl malonate (**III**) in presence of appropriate base *(e.g.NaH, NaOH, KOH, KOt-Bu and the like)* in an appropriate solvent such as THF, DMF, toluene, dioxane, dimethyl sulfoxide and the like or their suitable mixtures. The reaction may be carried out at the temperatures ranging from 20 °C to the boiling point of the solvent(s) used. The reaction may be carried out in an inert atmosphere. The reaction time may range from 2 hours to 2 days.

**Method B:** Compounds of formula (**V**) wherein 'HET' and 'X' are as defined earlier may be prepared by the reduction of compounds of general formula (**IV**) where 'HET' and 'X' are as defined earlier with appropriate reducing agents such as LiAlH₄, NaBH₄ and the like in an appropriate solvent such as THF, diethyl ether, dioxane, ethanol, methanol, water and the like or their suitable mixtures. The reaction may be carried out at the temperatures ranging from 20 °C to the boiling point of the solvent(s) used. The reaction may be carried out in an inert atmosphere. The reaction time may range from 2 hours to 2 days.

**Method C:** The diol of formula (V) may be converted to dioxane of formula (**VII**) wherein 'HET', 'X' and R₁ are as defined earlier and R₆ is C₍₁₋₆₎ linear or branched alkyl, cycloalkyl or aryl group as defined earlier, by reacting with appropriate ketoester R₁C(O)COOR₆ (**VI**) in presence of a suitable Lewis acid e.g. boron trifluoride etherate complex and the like. Reaction may be conducted in an appropriate solvent e.g., polar solvents such as acetonitrile or N,N-dimethyl formamide (DMF); ether solvents such as tetrahydrofuran (THF) or diethyl ether, diisopropyl ether 1,2-dimethoxyethane; halogenated hydrocarbon solvents such as chloroform or dichloromethane; hydrocarbon solvents such as benzene, toluene, hexane, heptane or mixtures of appropriate solvents selected from those described above. The reaction may be carried out at a temperature in the range -20 °C to reflux temperature of the solvent(s) used and the reaction time may range from 1 h to 4 days.

**Method D:** The compound of formula (**VII**) may be hydrolysed to compound of formula (**VIII**) where 'HET', R₁ and 'X' are as defined earlier using suitable base e.g., NaOH, LiOH, KOH and the like. Reaction may be conducted in suitable solvents such as alcohols like methanol, ethanol, propanol, isopropanol, butanol and the like, THF, water or mixtures thereof. The reaction may be carried out at a temperature in the range 20 °C to reflux temperature of the solvent(s) used and the reaction time may range from 1 to 48 hours.

**Method E:** Compounds of formula **(Ia)** where 'HET', R₁, 'X' and 'Y' are as defined earlier may be prepared by coupling of compounds of formula **(VIII)** and YH using suitable methods available in the literature for standard peptide coupling.

**Method F:** Compounds of formula **(I)** wherein 'Y' is NR₂R₃ & where R₂ is OH and 'HET', R₁, R₃ and 'X' are as defined earlier may be prepared by debenzylation of compounds of formula **(Ia)** where 'Y' is NR₂R₃ wherein R₂ is OBn and 'HET', R₁, R₃ and 'X' are as defined earlier using suitable methods available in the literature for deprotection of a benzyl group.

**Method G:** Compounds of formula **(I)** where 'Y' is NR₂R₃ wherein R₂ is OH and 'A', R₁, R₃ and 'X' are as defined earlier may also be prepared from corresponding acid compounds of formula **(VIII)** using suitable methods available in the literature for conversion of acid to hydroxamic acid.

**Method H:** Compounds of formula **(I)** where 'Y' is NR₂R₃ & wherein R₂ is OH and 'A', R₁, R₃ and 'X' are as defined earlier may further be prepared from corresponding ester compounds of formula **(VII)** using suitable methods available in the literature for conversion of an ester to a hydroxamic acid.

**Scheme 2:** The compounds of general formula **(Ia)** wherein 'HET' represents hetereoaryl or heterocyclyl group which are connected to X through N atom, may also be prepared by reactions outlined in **Scheme 2** below. 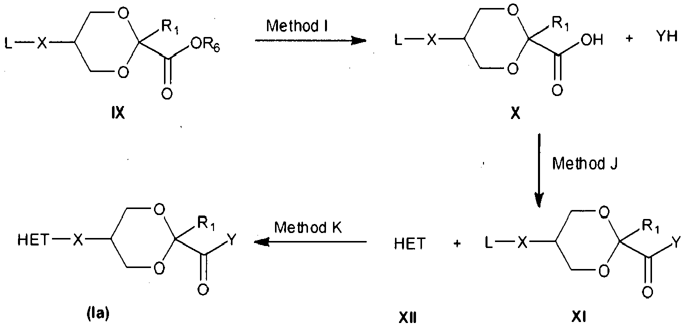 **Method I:** The compound of formula **(IX)** may be hydrolyzed to compound of formula **(X)** wherein 'L', 'X', R₁ and R₆ are as defined earlier using suitable base e.g., NaOH, LiOH, KOH and the like. Reaction may be conducted in suitable solvents such as alcohols like methanol, ethanol, propanol, isopropanol, butanol and the like, THF, water or mixtures thereof. The reaction may be carried out at a temperature in the range 20 °C to reflux temperature of the solvent(s) used and the reaction time may range from 1 to 48 hours.

**Method J:** Compounds of formula **(XI)** wherein R₁, 'L', 'X' and 'Y' are as defined earlier may be prepared by coupling of compounds of formula **(X)** and YH using suitable methods available in the literature for standard peptide coupling.

**Method K:** Compounds of formula **(Ia)** wherein 'HET' represents heteroaryl or heterocyclyl group and R₁, 'X' and 'Y' are as defined earlier may be prepared by reacting compounds of general formula **(XI)** wherein R₁, 'L', 'X' and 'Y' are as defined earlier with compounds of general formula **(XII)** wherein 'HET' represents heteroaryl or heterocyclyl group in presence of appropriate base *(e.g .NaOH, KOH, NaH, KOt-Bu and the like)* in an appropriate solvent such as dimethyl sulfoxide, THF, DMF, toluene, dioxane and the like or their suitable mixtures. The reaction may be carried out at the temperatures ranging from 0 °C to the boiling point of the solvent(s) used. The reaction may be carried out in an inert atmosphere. The reaction time may range from 2 hours to 2 days.

The compounds of formula (I) or pharmaceutical compositions containing them are useful as PCSK9 ligands suitable for humans and other warm blooded animals, and may be administered either by oral, topical or parenteral administration for the treatment of various disease conditions associated with dyslipidemia, and related disorders.

The pharmaceutical composition is provided by employing conventional techniques. Preferably the composition is in unit dosage form containing an effective amount of the active component, that is, the compounds of formula (I) according to this invention.

The quantity of active component, that is, the compounds of formula (I) according to this invention, in the pharmaceutical composition and unit dosage form thereof may be varied or adjusted widely depending upon the particular application method, the potency of the particular compound and the desired concentration. Generally, the quantity of active component will range between 0.5% to 90% by weight of the composition.

The compounds of the invention or their suitable pharmaceutical compositions are inhibitors of PCSK9 & can be used for the treatment of dyslipidemia and related diseases to an individual in need of such treatment.

The invention is explained in greater detail by the examples given below, which are provided by way of illustration only and therefore should not be construed to limit the scope of the invention as is otherwise described elsewhere in the specification. *¹H NMR spectral data given in the examples (vide infra) are recorded using a 400 MHz spectrometer (Bruker AVANCE-400) and reported in δ scale. Until and otherwise mentioned the solvent used for NMR is CDCl₃ using tetramethyl silane as the internal standard. Mass (ESI-MS) spectral data in the examples are recorded using a Shimadzu LC-MS 2010-A spectrometer.*

### Example 1

### N-(benzyloxy)-2-methyl-5-c-((5-methyl-2-phenyloxazol-4-yl)methyl)-1,3-dioxane-2-carboxamide

### Step 1: diethyl 2-((5-methyl-2-phenyloxazol-4-yl)methyl)malonate

Diethyl malonate (58 gm, 0.12 moles) was added dropwise to an ice cold suspention of NaH (50%) (8.7 gm, 0.18 moles) in THF (50 ml) at 0-10 °C over a period of 30 min. under nitrogen atmosphere and reaction mixture was stirred at about 25 °C for 30 min. A solution of 4-(chloromethyl)-5-methyl-2-phenyloxazole (25 gm, 0.12 moles) in THF (100 ml) was added to the reaction mixture at 25 °C and reaction mixture was stirred at 25 °C for 18 hours. The reaction mixture was poured into ice cold water and extracted by ethyl acetate. The combined ethyl acetate extract was washed with water and brine, dried over sodium sulphate and evapourated in vacuum. Excess diethyl malonate was distilled out under vacuum and the residue was purified by column chromatography (Eluent : 5% to 7% ethyl acetate in hexane) to yield 23 gm required product as thick liquid.
¹H NMR: 1.24 (t, *J* = 7.2 Hz, 6H), 2.34 (s, 3H), 3.07 (d, *J* = 7.6 Hz, 2H), 3.88 (t, *J* = 7.6 Hz, 1H), 4.15-4.22 (m, 2H), 7.39-7.45 (m, 3H), 7.94-7.97 (m, 2H).
ESI/MS m/z : 331.1 (M+H)⁺
Yield: 58%

### Step 2: 2-((5-methyl-2-phenyloxazol-4-yl)methyl)propane-1,3-diol

NaBH₄ (21 gm, 0.56 moles) was added in small portions to a solution of the product of step 1 (23 gm, 0.69 moles) in EtOH (80 ml) at 10 °C and the reaction mixture was stirred at 25 °C for 6 hours. The reaction mixture was poured into ice cold water, acidified (2 pH) and extracted with ethyl acetate. The combined ethyl acetate extract was washed with water and brine, dried over sodium sulphate and evapourated in vacuum. The residue was triturated in diisopropyl ether to yield 8.0 gm of product as white solid.
¹H NMR: 2.03-2.11 (m, 1H), 2.36 (s, 3H), 2.66 (d, *J* = 6.8 Hz, 6H), 3.71-3.82 (m, 4H), 7.39-7.44 (m, 3H), 7.94-7.98 (m, 2H).
ESI/MS m/z : 247.1 (M+H)⁺
Yield: 46%

### Step 3: methyl 2-methyl-5-((5-methyl-2-phenyloxazol-4-yl)methyl)-1,3-dioxane-2-carboxylate

Methyl pyruvate (15.5 ml, 0.17 moles) was added to a solution of the product of step 2 (7.0 gm, 0.028 moles) in CH₃CN (50 ml) followed by drop wise addition of BF₃.Et₂O (10.68 ml, 0.085 moles) at 25 °C under nitrogen atmosphere and the reaction mixture was stirred at 25 °C for 18 hours. The reaction mixture was poured into ice cold NaHCO₃ solution (300 ml) and extracted by ethyl acetate. The combined ethyl acetate extract was washed with water and brine, dried over sodium sulphate and evapourated in vacuum. The crude product was purified by column chromatography (Eluent : 15% ethyl acetate in hexane) to yield 6.5 gm required product as thick liquid.
ESI/MS m/z : 331.1 (M+H)⁺
Yield: 69%

### Step 4: 2-methyl-5-((5-methyl-2-phenyloxazol-4-yl)methyl)-1,3-dioxane-2-carboxylic acid

To a solution of the product of step 3 (5.0 gm, 15.10 mmoles) in a mixture of methanol (6 ml), THF (18 ml) and H₂O (6 ml) was added a solution of lithium hydroxide (1.27 gm, 30.20 mmoles) in water and the reaction mixture was stirred at ambient temperature for 3 hours. The solvents were evaporated and the residue was dissolved in water, acidified with 1N HCl and extracted with ethyl acetate. The combined ethyl acetate extract was washed with water and brine, dried over sodium sulphate and evaporated under reduced pressure to yield 3.5 gm of product as the mixture of *cis* and *trans* isomer as white solid.
ESI/MS m/z : 317.1 (M+H)⁺
Yield: 77%

### Step 5: N-(benzyloxy)-2-methyl-5-((5-methyl-2-phenyloxazol-4-yl)methyl)-1,3-dioxane-2-carboxamide

To a solution of the product of step 4 (1.76 gm, 5.6 mmoles) in DMF (10 mL), O-benzyl hydroxylamine hydrochloride (927 mg, 5.8 mmoles), HOBT (1.16 gm, 8.7 mmoles), EDCI (1.28 gm, 6.6 mmoles) and N-ethyl morpholine (2.11 ml, 16.7 mmoles) were added and reaction mixture was srirred at 25 °C for 5 hours under nitrogen atmosphere. The reaction mixture was poured into ice cold water. White solid seperated which was filtered and washed with water & dried over P₂O₅ under vacuum to yield 1.7 gm of the desired product as thick liquid.
ESI/MS m/z : 445.0 (M+H)⁺
Yield: 86%

### Example 2

### N-(benzyloxy)-2-methyl-5-c-((5-methyl-2-phenyloxazol-4-yl)methyl)-1,3-dioxane-2-carboxamide

### Step 1: 2-methyl-5-t-((5-methyl-2-phenyloxazol-4-yl)methyl)-1,3-dioxane-2-carboxylic acid

The product obtained from step 4 of example 1 was recrystallized using 5% methanol in ethyl acetate (20 ml) at 25 °C to yield 1.76 mg of pure cis isomer as white solid.
¹H NMR (DMSO-D₆): 1.32 (s, 3H), 2.16-2.24 (m, 3H), 2.28 (m, 3H), 3.44 (t, *J* = 11.2 Hz, 2H), 3.82 (dd, *J* = 11.4 & 3.4 Hz, 2H), 7.45-7.51 (m, 3H), 8.87-8.90 (m, 2H).
ESI/MS m/z : 317.2 (M+H)⁺
Yield: 37%

### Step 2: N-(benzyloxy)-2-methyl-5-c-((5-methyl-2-phenyloxazol-4-yl)methyl)-1,3-dioxane-2-carboxamide

To a solution of the product of step 1 (1.76 gm, 5.6 mmoles) in DMF (10 mL), O-benzyl hydroxylamine hydrochloride (927 mg, 5.8 mmoles), HOBT (1.16 gm, 8.7 mmoles), EDCI (1.28 gm, 6.6 mmoles) and N-ethyl morpholine (2.11 ml, 16.7 mmoles) were added and reaction mixture was srirred at 25 °C for 5 hours under nitrogen atmosphere. The reaction mixture was poured into ice cold water. White solid seperated which was filtered and washed with water & dried over P₂O₅ under vacuum to yield 1.7 gm of the desired product as thick liquid.
¹H NMR (DMSO-D₆): 1.25 (s, 3H), 2.11-2.15 (m, 3H), 2.28 (s, 3H), 3.30 (t, J = 11.0 Hz, 2H), 3.74-3.78 (m, 2H), 4.81 (s, 2H), 7.32-7.39 (m, 5H), 7.45-7.49 (m, 3H), 7.50-7.89 (m, 2H), 11.45 (s, NH).
ESI/MS m/z : 423.2 (M+H)⁺
Yield: 45%

### Example 3

### N-(benzyloxy)-2-methyl-5-t-((5-methyl-2-phenyloxazol-4-yl)methyl)-1,3-dioxane-2-carboxamide

### Step 1: 2-methyl-5-t-((5-methyl-2-phenyloxazol-4-yl)methyl)-1,3-dioxane-2-carboxylic acid

The mother liquor after the crystallization of cis isomer in step 1 of example 2 was concentrated under vacuum. The residue was recrystallized using ethyl acetate (30 ml) at 25 °C to yield 1.5 gm of pure trans isomer as off white solid.
¹H NMR (DMSO-D₆): 1.39 (s, 3H), 1.70-1.74 (m, 1H), 2.23 (s, 3H), 2.76 (d, J = 7.6 Hz, 2H), 3.63 (d, J = 11.2 Hz, 2H), 3.88 (d, J = 11.8 & 2.2 Hz, 2H), 7.43-7.51 (m, 3H), 8.86-8.89 (m, 2H).
ESI/MS m/z : 317.0 (M+H)⁺
Yield: 31%

### Step 2: N-(benzyloxy)-2-methyl-5-t-((5-methyl-2-phentloxazol-4-yl)methyl)-1,3-dioxane-2-carboxamide

To a solution of the product of step 4 (1.50 gm, 4.77 mmoles) in DMF (10 mL), O-benzyl hydroxylamine hydrochloride (927 mg, 5.8 mmoles), HOBT (1.00 gm, 7.44 mmoles), EDCI (1.10 gm, 5.7 mmoles) and N-ethyl morpholine (1.80 ml, 14.31 mmoles) were added and reaction mixture was srirred at 25 °C for 5 hours under nitrogen atmosphere. The reaction mixture was poured into ice cold water. White solid seperated which was filtered and washed with water & dried over P₂O₅ under vacuum to yield 1.4 gm of the desired product as thick liquid.
¹H NMR : 1.49 (s, 3H), 1.83-1.89 (m, 1H), 2.34 (s, 3H), 2.78 (d, J = 7.6 Hz, 2H), 3.67 (d, J = 11.2 Hz, 2H), 3.85 (dd, J = 11.8 Hz, 2H), 4.99 (s, 2H), 7.34-7.45 (m, 8H), 7.94-7.97 (m, 2H), 8.67 (s, NH).
ESI/MS m/z : 423.2 (M+H)⁺
Yield: 42%

### Example 4

### N-(Hydroxy)-2-methyl-5-((5-methyl-2-phenyloxazol-4-yl)methyl)-1,3-dioxane-2-carboxamide

To a suspension of 10% palladium on charcoal (150 mg) in methanol (20 mL) was added the product of step 5 (1.7 gm, 4.04 mmoles) followed by addition of ammonium formate (1.27 g, 20.2 mmoles) and the reaction mixture was heated to reflux for 2-5 hours. The reaction mixture was cooled to ambient temperature and the catalyst was filtered off. The filtrate was evaporated and the residue was taken in ethyl acetate and washed with water. The organic phase was dried over sodium sulfate and evaporated under reduced pressure. The product was recrystallised from a mixture of ethyl acetate and petroleum ether (1:1) (25 ml) to obtain 900 mg of the desired product as white solid.
ESI/MS m/z : 333.1 (M+H)⁺
Yield: 67%

### Example 5

### N-benzyloxy-5-c-(3-(9H-carbazol-9-yl)propyl)-2-methyl-1,3-dioxane-2-carboxamide

### Step 1: c-5-(3-chloropropyl)-2-methyl-1,3-dioxane-2-carboxylic acid

To a solution of methyl 5-(3-chloropropyl)-2-methyl-1,3-dioxane-2-carboxylate (5.0 gm, 0.021 moles) in a mixture of methanol (8 ml), THF (24 ml) and H₂O (8 ml) was added a solution of lithium hydroxide (1.77 gm, 0.042 moles) in water and the reaction mixture was stirred at ambient temperature for 18 hours. The solvents were evaporated and the residue was dissolved in water, acidified with 1N HCl and extracted with ethyl acetate. The ethyl acetate extract was washed with water and brine, dried over sodium sulphate and evaporated under reduced pressure to yield 3.91 gm of product as thick liqid.
¹H NMR: 1.20-1.27 (m, 2H), 1.58 (s, 3H), 1.71-1.78 (m, 2H), 2.03-2.09 (m, 1H), 3.47-3.59 (m, 4H), 3.98 (dd, *J*= 12.2 & 4.6 Hz, 2H).
ESI/MS m/z : 222.1 (M+H)⁺

### Yield: 83%

### Step 2: N-(benzyloxy)-5-c-(3-chloropropyl)-2-methyl-1,3-dioxane-2-carboxamide

To a solution of the product of step 1 (3.91 gm, 0.0176 moles) in DMF (20 mL), *O*-benzyl hydroxylamine hydrochloride (2.93 gm, 0.0184 moles), HOBT (3.70 gm, 0.0275 moles), EDCI (4.05 gm, 0.0211 moles) and N-ethyl morpholine (6.2 ml, 0.0528 moles) were added and reaction mixture was srirred at 25 °C for 24 hours under nitrogen atmosphere. The reaction mixture was poured into ice cold water and extracted by ethyl acetae. The ethyl acetate extract was washed with water and brine, dried over sodium sulphate and evapourated under reduced pressure. The crude product was triturated in hexane to yield 5.3 gm of product as thick liquid.
¹H NMR: 1.12-1.20 (m, 2H), 1.45 (s, 3H), 1.79-1.82 (m, 2H), 1.89-1.94 (m, 1H), 3.27 (t, *j* = 11.4 Hz, 2H), 3.45-3.49 (m, 2H), 3.85-3.88 (m, 2H), 5.01 (s, 2H), 7.35-7.41 (m, 5H).
ESI/MS m/z : 327.1 (M+H)⁺

### Yield: 89%

### Step 3: N-(benzyloxy)-c-5-(3-(9H-carbazol-9-yl)propyl)-2-methyl-1,3-dioxane-2-carboxamide

To an ice cold suspension of KOH powder (685 mg, 12.23 mmoles) in DMSO (2 ml), a solution of the product of step 2 (1.0 gm, 3.06 mmoles) and 9H-carbazole (51 mg, 3.06 mmoles) in DMSO (3 mL) was added dropwise at 0-5 °C under nitrogen atmosphere and reaction mixture was srirred at 25 °C for 2 hours under nitrogen atmosphere. The reaction mixture was poured into ice cold water and extracted by ethyl acetae. The ethyl acetate extract was washed with water and brine, dried over sodium sulphate and evapourated under reduced pressure. The crude product was purified by column chromatography (Eluent: 17% ethyl acetate in hexane) to yield 500 mg of product as thick liquid.
¹H NMR: 1.02-1.04 (m, 2H), 1.39 (s, 3H), 1.79-1.83 (m, 2H), 1.89-1.91 (m, 1H), 3.15 (t, J = 11.6 Hz, 2H), 3.74 (dd, J = 11.8 & 4.2, 2H), 4.28 (t, J = 7.0 Hz, 2H), 4.96 (s, 2H), 7.22-7.26 (m, 2H), 7.34-7.44 (m, 7H), 7.45-7.49 (m, 2H), 8.11 (d, J = 7.6 Hz, 2H), 8.55 (s, NH)..
ESI/MS m/z : 459.1 (M+H)⁺
Yield: 92%

The following examples were prepared following the general procedures given in Example **1-5,** with suitable modifications, alterations and other process variations which are within the scope of a person skilled in the art.

### Example 6

### N-hydroxy-2-methyl-5-c-((5-methyl-2-phenyloxazol-4-yl)methyl)-1,3-dioxane-2-carboxamide

¹H NMR (DMSO-D₆): 1.28 (s, 3H), 2.12-2.18 (m, 1H), 2.20-2.21 (m, 2H), 2.28 (s, 3H), 3.43 (t, J = 11.0 Hz, 2H), 3.79-3.83 (dd, J = 12.0 & 4.0 Hz, 2H), 7.47-7.51 (m, 3H), 7.88-7.90 (m, 2H), 8.85 (s, NH), 10.84 (s, OH).
ESI/MS m/z : 333.2 (M+H)⁺
Yield: 67%

### Example 7

### N-hydroxy-2-methyl-5-t-((5-methyl-2-phenyloxazol-4-yl)methyl)-1,3-dioxane-2-carboxamide

¹H NMR (DMSO-D₆): 1.36 (s, 3H), 1.74 (m, 1H), 2.33 (s, 3H), 2.73 (d, J = 7.6 Hz, 2H), 3.60 (d, J = 11.2 Hz, 2H), 3.84 (d, J = 10.0 Hz, 2H), 7.47-7.51 (m, 3H), 7.88-7.90 (m, 2H), 8.83 (s, NH), 10.81 (s, OH).
ESI/MS m/z : 333.2 (M+H)⁺
Yield: 27%

### Example 8

### 2-methyl-5-((5-methyl-2-phenyloxazol-4-yl)methyl)-N-((tetrahydro-2H-pyran-4-yl)methyl)-1,3-dioxane-2-carboxamide

ESI/MS m/z : 415.1 (M+H)⁺
Yield: 76%

### Example 9

### 2-methyl-5-c-((5-methyl-2-phenyloxazol-4-yl)methyl)-N-((tetrahydro-2H-pyran-4-yl)methyl)-1,3-dioxane-2-carboxamide

¹H NMR (DMSO-D₆): 1.05-1.15 (m, 2H), 1.27 (s, 3H), 1.45-1.49 (m, 2H), 1.65-1.72 (m, 1H), 2.13-2.20 (m, 1H), 2.22 (d, J = 6.8 Hz, 2H), 2.27 (s, 3H), 2.96 (t, J =6.4 Hz, 2H), 3.17-3.22 (t, J = 9.6 H, 2H), 3.39 (t, J = 11.2 Hz, 2H), 3.77-3.84 (m, 4H), 7.44-7.51 (m, 3H), 7.86-7.90 (m, 2H), 8.05 (t, NH).
ESI/MS m/z : 416.1 (M+H)⁺
Yield: 95%

### Example 10

### 2-methyl-5-t-((5-methyl-2-phenyloxazol-4-yl)methyl)-N-((tetrahydro-2H-pyran-4-yl)methyl)-1,3-dioxane-2-carboxamide

ESI/MS m/z : 416.1 (M+H)⁺
Yield: 84%

### Example 11

### (2-methyl-5-((5-methyl-2-phenyloxazol-4-yl)methyl)-1,3-dioxan-2-yl)(morpholino)methanone

ESI/MS m/z : 387.2 (M+H)⁺
Yield: 41%

### Example 12

### (2-methyl-5-c-((5-methyl-2-phenyloxazol-4-yl)methyl)-1,3-dioxan-2-yl)(morpholino)methanone

¹H NMR(DMSO-D₆): 1.33 (s, 3H), 2.18-2.25 (m, 3H), 2.28 (s, 3H), 3.51-3.56 (m, 8H), 3.76-3.77 (m, 2H), 3.82 (dd, J = 11.6 & 3.6 Hz, 2H), 7.44-7.51 (m, 3H), 7.87-7.90 (m, 2H).
ESI/MS m/z : 409.0 (M+Na)⁺
Yield: 65%

### Example 13

### (2-methyl-5-t-((5-methyl-2-phenyloxazol-4-yl)methyl)-1,3-dioxan-2-yl)(morpholino)methanone

ESI/MS m/z : 387.2 (M+H)⁺
Yield: 58%

### Example 14

### (2-methyl-5-c-((5-methyl-2-phenyloxazol-4-yl)methyl)-1,3-dioxan-2-yl)(4-methylpiperazin-1-yl)methanone

¹H NMR: 1.49 (s, 3H), 2.21 (d, J = 7.2 Hz, 2H), 2.28 (s, 3H), 2.30 (s, 3H), 2.39-2.50 (m, 5H), 3.63 (t, J = 11.6 Hz, 2H), 3.72-3.74 (m, 2H), 3.86-3.88 (m, 2H), 3.96 (dd, J = 11.8 & 4.6 Hz, 2H), 7.39-7.45 (m, 3H), 7.95-7.97 (m, 2H).
ESI/MS m/z : 400.0 (M+H)⁺
Yield: 48%

### Example 15

### (2-methyl-5-c-((5-methyl-2-phenyloxazol-4-yl)methyl)-1,3-dioxan-2-yl)(piperidin-1-yl)methanone

¹H NMR: 1.49 (s, 3H), 1.53-1.73 (m, 6H), 2.20 (d, J = 7.2 Hz, 2H), 2.28 (s, 3H), 2.42-2.51 (m, 1H), 3.62-3.68 (m, 4H), 3.78-3.79 (m, 2H), 3.96 (dd, J = 11.8 & 4.6 Hz, 2H), 7.38-7.45 (m, 3H), 7.95-7.97 (m, 2H).
ESI/MS m/z : 384.9 (M+H)⁺
Yield: 85%

### Example 16

### (2-methyl-5-c-((5-methyl-2-phenyloxazol-4-yl)methyl)-1,3-dioxan-2-yl)(thiomorpholino)methanone

¹H NMR: 1.50 (s, 3H), 2.21 (d, J = 7.2 Hz, 2H), 2.28 (s, 3H), 2.43-2.49 (m, 1H), 2.66-2.68 (m, 4H), 3.63 (t, J = 11.4 Hz, 2H), 3.94-3.95 (m, 2H), 3.97 (dd, J = 11.8 & 4.6 Hz, 2H), 4.10-4.11 (m, 2H), 7.40-7.45 (m, 3H), 7.95-7.97 (m, 2H).
ESI/MS m/z : 403.3 (M+H)⁺
Yield: 84%

### Example 17

### (1,1-dioxidothiomorpholino)(2-methyl-5-c-((5-methyl-2-phenyloxazol-4-yl)methyl)-1,3-dioxan-2-yl)methanone

¹H NMR: 1.53 (s, 3H), 2.24 (d, J = 7.2 Hz, 2H), 2.29 (s, 3H), 2.43-2.49 (m, 1H), 3.063.08 (m, 4H), 3.60 (t, J = 11.6 Hz, 2H), 4.05 (dd, J = 11.8 & 4.6 Hz, 2H), 4.19-4.20 (m, 2H), 4.36 (bs, 2H), 7.41-7.46 (m, 3H), 7.94-7.97 (m, 2H).
ESI/MS m/z : 434.8 (M+H)⁺
Yield: 74%

### Example 18

### 2-methyl-5-((5-methyl-2-phenyloxazol-4-yl)methyl)-N-(2-morpholino-2-oxoethyl)-1,3-dioxane-2-carboxamide

ESI/MS m/z : 444.1 (M+H)⁺
Yield: 55%

### Example 19

### 2-methyl-5-c-((5-methyl-2-phenyloxazol-4-yl)methyl)-N-(2-morpholino-2-oxoethyl)-1,3-dioxane-2-carboxamide

¹H NMR: 1.51 (s, 3H), 2.25 (d, J = 7.2 Hz, 2H), 2.28 (s, 3H), 2.38-2.45 (m, 1H), 3.42 (t, J = 4.8 Hz, 2H), 3.57-3.66 (m, 4H), 3.69-3.72 (m, 4H), 4.01 (dd, J = 12.0 & 4.4 Hz, 2H), 4.13 (d, J = 4.4 Hz, 2H), 7.32-7.33 (m, NH), .7.39-7.44 (m, 3H), 7.94-7.96 (m, 2H).
ESI/MS m/z : 444.3 (M+H)⁺
Yield: 84%

### Example 20

### 2-methyl-5-t-((5-methyl-2-phenyloxazol-4-yl)methyl)-N-(2-morpholino-2-oxoethyl)-1,3-dioxane-2-carboxamide

ESI/MS m/z : 444.1 (M+H)⁺
Yield: 49%

### Example 21

### 2-methyl-5-c-((5-methyl-2-phenyloxazol-4-yl)methyl)-N-(2-(4-methylpiperazin-1-yl)-2-oxoethyl)-1,3-dioxane-2-carboxamide

¹H NMR: 1.50 (s, 3H), 2.24 (d, J = 7.2 Hz, 2H), 2.28 (s, 3H), 2.32 (s, 3H), 2.37-2.44 (m, 5H), 3.42 (t, J = 5.0 Hz, 2H), 3.56 (t, J = 11.4 Hz, 2H), 3.65 (t, J = 5.0 Hz, 2H), 4.00 (dd, J = 12.0 & 4.4 Hz, 2H), 4.13 (d, J = 4.0 Hz, 2H), 7.34-7.36 (m, NH), 7.37-7.44 (m, 3H), 7.93-7.96 (m, 2H).
ESI/MS m/z : 457.5 (M+H)⁺
Yield: 52%

### Example 22

### 2-methyl-5-c-((5-methyl-2-phenyloxazol-4-yl)methyl)-N-(2-oxo-2-(piperidin-1-yl)ethyl)-1,3-dioxane-2-carboxamide

¹H NMR: 1.51 (s, 3H), 1.55-1.71 (m, 6H), 2.24 (d, J = 7.6 Hz, 2H), 2.28 (s, 3H), 2.37-2.44 (m, 1H), 3.34 (t, J = 5.4 Hz, 2H), 3.56-3.62 (m, 4H), 4.00 (dd, J = 12.0 & 4.4 Hz, 2H), 4.12 (d, J = 5.2 Hz, 2H), 7.39-7.44 (m, 4H), 7.93-7.96 (m, 2H).
ESI/MS m/z : 442.4 (M+H)⁺
Yield: 68%

### Example 23

### 2-methyl-5-c-((5-methyl-2-phenyloxazol-4-yl)methyl)-N-(2-oxo-2-thiomorpholinoethyl)-1,3-dioxane-2-carboxamide

¹H NMR: 1.51 (s, 3H), 2.25 (d, J = 7.6 Hz, 2H), 2.28 (s, 3H), 2.37-2.45 (m, 1H), 2.64-2.66 (m, 4H), 3.56 (t, J = 11.2 Hz, 2H), 3.68 (t, J = 5.0 Hz, 2H), 3.90 (t, J = 5.0 Hz, 2H), 4.01 (dd, J = 12.0 & 4.4 Hz, 2H), 4.13 (d, J = 4.4 Hz, 2H), 7.34 (s, NH), 7.38-7.45 (m, 3H), 7.94-7.97 (m, 2H).
ESI/MS m/z : 460.4 (M+H)⁺
Yield: 54%

### Example 24

### N-(2-(1,1-dioxidothiomorpholino)-2-oxoethyl)-2-methyl-5-c-((5-methyl-2-phenyloxazol-4-yl)methyl)-1,3-dioxane-2-carboxamide

¹H NMR: 1.51 (s, 3H), 2.27-2.29 (m, 5H), 2.37-2.43 (m, 1H), 3.07-3.10 (m, 4H), 3.58 (t, J = 11.0 Hz, 2H), 2.942.96 (m, 2H), 4.02 (dd, J = 12.0 & 4.0 Hz, 2H), 4.13-4.14 (m, 2H), 4.20 (d, J = 4.8 Hz, 2H), 7.21 (bs, NH), 7.41-7.44 (m, 3H), 7.94-7.96 (m, 2H).
ESI/MS m/z : 491.8 (M+H)⁺
Yield: 47%

### Example 25

### 2-methyl-5-c-((5-methyl-2-phenyloxazol-4-yl)methyl)-N-(2-oxo-2-(((tetrahydro-2H-pyran-4-yl)methyl)amino)ethyl)-1,3-dioxane-2-carboxamide

¹H NMR: 1.25-1.32 (m, 2H), 1.50 (s, 3H), 1.59-1.63 (m, 2H), 1.71-1.80 (m, 1H), 2.26-2.29 (m, 5H), 2.35-2.45 (m, 1H), 3.17 (t, J = 6.4 Hz, 2H), 3.33-3.39 (m, 2H), 3.54 (t, J = 11.0 Hz, 2H), 3.95-4.05 (m, 6H), 6.12 (s, NH), 7.08 (t, J = 5.4 Hz, NH), 7.38-7.45 (m, 3H), 7.94-7.97 (m, 2H).
ESI/MS m/z : 427.0 (M+H)⁺
Yield: 53%

### Example 26

### 2-methyl-5-c-((5-methyl-2-phenyloxazol-4-yl)methyl)-N-(3-morpholino-3-oxopropyl)-1,3-dioxane-2-carboxamide

¹H NMR: 1.45 (s, 3H), 2.25 (d, J = 7.2 Hz, 2H), 2.29 (s, 3H), 2.33-2.39 (m, 1H), 2.54 (t, J = 5.8 Hz, 2H), 3.44 (t, J = 4.8 Hz, 2H), 3.50 (t, J = 11.0 Hz, 2H), 3.62-3.68 (m, 8H), 3.98 (dd, J = 12.0 & 4.4 Hz, 2H), 7.13 (t, J = 6.2 Hz, NH), 7.40-7.45 (m, 3H), 7.94-7.96 (m, 2H).
ESI/MS m/z : 458.0 (M+H)⁺
Yield: 84%

### Example 27

### 2-methyl-5-c-((5-methyl-2-phenyloxazol-4-yl)methyl)-N-(3-oxo-3-(((tetrahydro-2H-pyran-4-yl)methyl)amino)propyl)-1,3-dioxane-2-carboxamide

¹H NMR: 1.25-1.35 (m, 2H), 1.45 (s, 3H), 1.501.51 (m, 2H), 1.69-1.78 (m, 1H), 2.25 (d, J = 7.2 Hz, 2H), 2.29 (s, 3H), 2.33-2.39 (m, 1H), 2.43 (t, J = 6.0 Hz, 2H), 3.14 (t, J = 6.4 Hz, 2H), 3.31 (t, J = 11.8 Hz, 2H), 3.50 (t, J = 10.6 Hz, 2H), 3.59 (q, J = 6.0 Hz, 2H), 3.94-4.02 (m, 4H), 5.82 (bs, NH), 7.06 (t, J = 6.2 Hz, NH), 7.40-7.45 (m, 3H), 7.94-7.96 (m, 2H).
ESI/MS m/z : 486.1 (M+H)⁺
Yield: 48%

### Example 28

### 2-methyl-5-c-((5-methyl-2-phenyloxazol-4-yl)methyl)-N-(3-oxo-3-thiomorpholinopropyl)-1,3-dioxane-2-carboxamide

¹H NMR: 1.45 (s, 3H), 2.24 (d, J = 7.6 Hz, 2H), 2.28 (s, 3H), 2.33-2.42 (m, 1H), 2.54 (t, J = 5.8 Hz, 2H), 2.59-2.62 (m, 4H), 3.49 (t, J = 11.2 Hz, 2H), 3.61 (q, J = 6.2 Hz, 2H), 3.70-3.75 (m, 2H), 3.85-3.92 (m, 2H), 3.98 (dd, J = 11.8 & 4.2 Hz, 2H), 7.12 (t, J = 6.2 Hz, NH), 7.38-7.45 (m, 3H), 7.94-7.96 (m, 2H).
ESI/MS m/z : 473.9 (M+H)⁺
Yield: 82%

### Example 29

### N-(3-(1,1-dioxidothiomorpholino)-3-oxopropyl)-2-methyl-5-c-((5-methyl-2-phenyloxazol-4-yl)methyl)-1,3-dioxane-2-carboxamide

¹H NMR: 1.45 (s, 3H), 2.28-2.36 (m, 6H), 2.64 (t, J = 6.0 Hz, 2H), 3.05-3.10 (m, 4H), 3.51 (t, J = 10.6 Hz, 2H), 3.61 (q, J = 6.0 Hz, 2H), 3.97-4.03 (m, 4H), 4.11-4.12 (m, 2H), 7.01 (t, J = 6.2 Hz, NH), 7.40-7.45 (m, 3H), 7.94-7.96 (m, 2H).
ESI/MS m/z : 506.1 (M+H)⁺
Yield: 23%

### Example 30

### 2-methyl-5-c-((5-methyl-2-phenyloxazol-4-yl)methyl)-N-(3-oxo-3-(piperidin-1-yl)propyl)-1,3-dioxane-2-carboxamide

¹H NMR: 1.45 (s, 3H), 1.50-1.56 (m, 4H), 1.60-1.64 (m, 2H), 2.23 (d, J = 7.2 Hz, 2H), 2.28 (s, 3H), 2.32-2.42 (m, 1H), 2.53 (t, J = 5.8 Hz, 2H), 3.36 (t, J = 5.4 Hz, 2H), 3.59-3.65 (m, 4H), 3.61 (q, J = 6.0 Hz, 2H), 3.97 (dd, J = 11.8 & 4.2 Hz, 2H), 7.20 (t, J = 6.2 Hz, NH), 7.37-7.44 (m, 3H), 7.94-7.97 (m, 2H).
ESI/MS m/z : 456.0 (M+H)⁺
Yield: 82%

### Example 31

### 2-methyl-5-c-((5-methyl-2-phenyloxazol-4-yl)methyl)-N-(3-(4-methylpiperazin-1-yl)-3-oxopropyl)-1,3-dioxane-2-carboxamide

¹H NMR: 1.45 (s, 3H), 2.24 (d, J = 7.2 Hz, 2H), 2.28 (s, 3H), 2.30 (s, 3H), 2.32-2.40 (m, 5H), 2.55 (t, J = 5.8 Hz, 2H), 3.45 (t, J = 5.0 Hz, 2H), 3.48-3.55 (m, 2H), 3.61-3.65 (m, 4H), 3.98 (dd, J = 12.0 & 4.4 Hz, 2H), 7.16 (t, J = 6.2 Hz, NH), 7.39-7.45 (m, 3H), 7.93-7.96 (m, 2H).
ESI/MS m/z : 471.0 (M+H)⁺
Yield: 63%

### Example 32

### 2-methyl-5-((5-methyl-2-phenyloxazol-4-yl)methyl)-N-(3-oxo-1-phenylbutan-2-yl)-1,3-dioxane-2-carboxamide

ESI/MS m/z : 463.5 (M+H)⁺
Yield: 77%

### Example 33

### N-benzyloxy-2-methyl-5-(2-(5-methyl-2-phenyloxazol-4-yl)ethyl)-1,3-dioxane-2-carboxamide

ESI/MS m/z : 437.2 (M+H)⁺
Yield: 52%

### Example 34

### N-hydroxy-2-methyl-5-(2-(5-methyl-2-phenyloxazol-4-yl)ethyl)-1,3-dioxane-2-carboxamide

ESI/MS m/z : 437.2 (M+H)⁺
Yield: 59%

### Example 35

### N-hydroxy-2-methyl-5-c-(2-(5-methyl-2-phenyloxazol-4-yl)ethyl)-1,3-dioxane-2-carboxamide

¹H NMR (CD₃OD): 1.37-1.44 (m, 5H), 1.96-1.99 (m, 1H), 2.35 (s, 3H), 2.50 (t, J = 7.6 Hz, 2H), 3.44 (t, J = 11.6 Hz, 2H), 3.91-3.96 (dd, J = 12 & 4.4 Hz, 2H), 7.45-7.49 (m, 3H), 7.93-7.96 (m, 2H).
ESI/MS m/z : 347.0 (M+H)⁺
Yield: 62%

### Example 36

### N-hydroxy-2-methyl-5-t-(2-(5-methyl-2-phenyloxazol-4-yl)ethyl)-1,3-dioxane-2-carboxamide

¹H NMR (CD₃OD): 1.44 (s, 3H), 1.48-1.49 (m, 1H), 1.97-2.03 (m, 2H), 2.36 (s, 3H), 2.59 (t, J = 7.6 Hz, 2H), 3.80 (d, J = 10.8 Hz, 2H), 3.95-3.98 (dd, J = 11.8 & 3.2 Hz, 2H), 7.46-7.49 (m, 3H), 7.94-7.96 (m, 2H).
ESI/MS m/z : 346.9 (M+H)⁺
Yield: 41%

### Example 37

### 2-methyl-5-(2-(5-methyl-2-phenyloxazol-4-yl)ethyl)-N-((tetrahydro-2H-pyran-4-yl)methyl)-1,3-dioxane-2-carboxamide

ESI/MS m/z : 429.3 (M+H)⁺
Yield: 98%

### Example 38

### 2-methyl-5-c-(2-(5-methyl-2-phenyloxazol-4-yl)ethyl)-N-((tetrahydro-2H-pyran-4-yl)methyl)-1,3-dioxane-2-carboxamide

¹H NMR: 1.25-1.45 (m, 4H), 1.47 (s, 3H), 1.58-1.62 (m, 2H), 1.74-1.82 (m, 1H), 2.01-2.07 (m, 1H), 2.31 (s, 3H), 2.43 (t, J = 7.8 Hz, 2H), 3.22 (t, J = 6.6 Hz, 2H), 3.34-3.41 (m, 2H), 3.42 (t, J = 11.4 Hz, 2H), 3.95 (dd, J = 12.0 & 4.4 Hz, 2H), 6.38 (t, J = 6.2 Hz, NH), 7.38-7.44 (m, 3H), 7.94-7.96 (m, 2H).
ESI/MS m/z : 429.4 (M+H)⁺
Yield: 90%

### Example 39

### 2-methyl-5-t-(2-(5-methyl-2-phenyloxazol-4-yl)ethyl)-N-((tetrahydro-2H-pyran-4-yl)methyl)-1,3-dioxane-2-carboxamide

ESI/MS m/z : 429.3 (M+H)⁺
Yield: 59%

### Example 40

### (2-methyl-5-(2-(5-methyl-2-phenyloxazol-4-yl)ethyl)-1,3-dioxan-2-yl)(morpholino)methanone

ESI/MS m/z : 401.2 (M+H)⁺
Yield: 98%

### Example 41

### (2-methyl-5-c-(2-(5-methyl-2-phenyloxazol-4-yl)ethyl)-1,3-dioxan-2-yl)(morpholino)methanone

¹H NMR: 1.39-1.45 (m, 2H), 1.49 (s, 3H), 2.04-2.12 (m, 1H), 2.31 (s, 3H), 2.42 (t, J = 7.8 Hz, 2H), 3.53 (t, J = 11.6 Hz, 2H), 3.64-3.77 (m, 6H), 3.87-3.89 (m, 2H), 3.94 (dd, J = 11.6 & 4.6 Hz, 2H), 7.37-7.44 (m, 3H), 7.94-7.97 (m, 2H).
ESI/MS m/z : 401.4 (M+H)⁺
Yield: 83%

### Example 42

### (2-methyl-5-t-(2-(5-methyl-2-phenyloxazol-4-yl)ethyl)-1,3-dioxan-2-yl)(morpholino)methanone

ESI/MS m/z : 401.3 (M+H)⁺
Yield: 79%

### Example 43

### (2-methyl-5-c-(2-(5-methyl-2-phenyloxazol-4-yl)ethyl)-1,3-dioxan-2-yl)(4-methylpiperazin-1-yl)methanone

¹H NMR: 1.40-1.46 (m, 2H), 1.50 (s, 3H), 2.05-2.11 (m, 1H), 2.30 (s, 3H), 2.32 (s, 3H), 2.38-2.46 (m, 6H), 3.54 (t, J = 11.6 Hz, 2H), 3.73-3.75 (m, 2H), 3.88-3.89 (m, 2H), 3.95 (dd, J = 12.0 & 4.8 Hz, 2H), 7.39-7.45 (m, 3H), 7.94-7.97 (m, 2H).
ESI/MS m/z : 413.9 (M+H)⁺
Yield: 80%

### Example 44

### (2-methyl-5-c-(2-(5-methyl-2-phenyloxazol-4-yl)ethyl)-1,3-dioxan-2-yl)(piperidin-1-yl)methanone

¹H NMR: 1.39 (q, J = 7.0 Hz, 2H), 1.49 (s, 3H), 1.55-1.64 (m, 6H), 2.02-2.11 (m, 1H), 2.31 (s, 3H), 2.42 (t, J = 7.8 Hz, 2H), 3.55-3.63 (m, 4H), 3.75 (t, J = 5.4 Hz, 2H), 3.93 (dd, J = 11.8 & 4.6 Hz, 2H), 7.37-7.44 (m, 3H), 7.94-7.97 (m, 2H).
ESI/MS m/z : 399.0 (M+H)⁺
Yield: 74%

### Example 45

### (2-methyl-5-c-(2-(5-methyl-2-phenyloxazol-4-yl)ethyl)-1,3-dioxan-2-yl)(thiomorpholino)methanone

¹H NMR: 1.39 (q, J = 7.2 Hz, 2H), 1.49 (s, 3H), 2.03-2.12 (m, 1H), 2.31 (s, 3H), 2.42 (t, J = 7.8 Hz, 2H), 2.62-2.69 (m, 4H), 3.52 (t, J = 11.6 Hz, 2H), 3.93-3.99 (m, 4H), 4.08-4.12 (m, 2H), 7.37-7.44 (m, 3H), 7.94-7.97 (m, 2H).
ESI/MS m/z : 438.9 (M+Na)⁺
Yield: 74%

### Example 46

### (1,1-dioxidothiomorpholino)(2-methyl-5-c-(2-(5-methyl-2-phenyloxazol-4-yl)ethyl)-1,3-dioxan-2-yl)methanone

¹H NMR: 1.40 (q, J = 7.2 Hz, 2H), 1.52 (s, 3H), 2.05-2.14 (m, 1H), 2.32 (s, 3H), 2.43 (t, J = 7.8 Hz, 2H), 3.04-3.10 (m, 4H), 3.46 (t, J = 11.4 Hz, 2H), 3.99 (dd, J = 11.8 & 4.6 Hz, 2H), 4.19-4.20 (m, 2H), 4.35-4.36 (m, 2H), 7.38-7.47 (m, 3H), 7.94-7.97 (m, 2H).
ESI/MS m/z : 449.0 (M+H)⁺
Yield: 80%

### Example 47

### 2-methyl-5-(2-(5-methyl-2-phenyloxazol-4-yl)ethyl)-N-(2-morpholino-2-oxoethyl)-1,3-dioxane-2-carboxamide

ESI/MS m/z : 459.0 (M+H)⁺
Yield: 75%

### Example 48

### 2-methyl-5-c-(2-(5-methyl-2-phenyloxazol-4-yl)ethyl)-N-(2-morpholino-2-oxoethyl)-1,3-dioxane-2-carboxamide

¹H NMR: 1.40 (q, J = 7.2 Hz, 2H), 1.50 (s, 3H), 2.03-2.09 (m, 1H), 2.31 (s, 3H), 2.42 (t, J = 7.6 Hz, 2H), 3.43-3.46 (m, 2H), 3.48 (t, J = 11.6 Hz, 2H), 3.64-3.67 (m, 2H), 3.70-3.73 (m, 4H), 3.98 (dd, J = 12.0 & 4.4 Hz, 2H), 4.14 (d, J = 4.4 Hz, 2H), 7.38-7.45 (m, 3H), 7.94-7.97 (m, 2H), 7.30 (bs, NH).
ESI/MS m/z : 459.1 (M+H)⁺
Yield: 68%

### Example 49

### 2-methyl-5-t-(2-(5-methyl-2-phenyloxazol-4-yl)ethyl)-N-(2-morpholino-2-oxoethyl)-1,3-dioxane-2-carboxamide

¹H NMR: 1.53 (s, 3H), 1.57-1.60 (m, 1H), 1.93 (q, J = 7.6 Hz, 2H), 2.32 (s, 3H), 2.52 (t, J = 7.6 Hz, 2H), 3.42 (m, 2H), 3.63 (m, 2H), 3.68 (m, 4H), 3.80 (dd, J = 11.8 & 4.2 Hz, 2H), 3.99 (d, J = 11.8 & 3.4 Hz, 2H), 4.11 (d, J = 4.4 Hz, 2H), 7.37-7.44 (m, 3H), 7.95-3.98 (m, 2H).
ESI/MS m/z : 458.1 (M+H)⁺
Yield: 72%

### Example 50

### 2-methyl-5-c-(2-(5-methyl-2-phenyloxazol-4-yl)ethyl)-N-(2-oxo-2-(((tetrahydro-2H-pyran-4-yl)methyl)amino)ethyl)-1,3-dioxane-2-carboxamide

¹H NMR: 1.25-1.36 (m, 2H), 1.40 (q, J = 7.2 Hz, 2H), 1.48 (s, 3H), 1.58 (dd, J = 13.0 & 1.6 Hz, 2H), 1.71-1.75 (m, 1H), 2.01-2.07 (m, 1H), 2.31 (s, 3H), 2.42 (t, J = 7.6 Hz, 2H), 3.17 (t, J = 6.0 Hz, 2H), 3.31-3.38 (m, 2H), 3.44 (t, J = 11.4 Hz, 2H), 3.94-4.01 (m, 6H), 6.29 (bs, NH), 7.08 (t, J = 6.4 Hz, NH), 7.39-7.44 (m, 3H), 7.93-7.96 (m, 2H).
ESI/MS m/z : 486.0 (M+H)⁺
Yield: 50%

### Example 51

### 2-methyl-5-c-(2-(5-methyl-2-phenyloxazol-4-yl)ethyl)-N-(2-(4-methylpiperazin-1-yl)-2-oxoethyl)-1,3-dioxane-2-carboxamide

¹H NMR: 1.39 (q, J = 7.4 Hz, 2H), 1.49 (s, 3H), 2.02-2.09 (m, 1H), 2.30 (s, 3H), 2.32 (s, 3H), 2.40-2.45 (m, 6H), 3.43 (t, J = 5.0 Hz, 2H), 3.47 (t, J = 11.6 Hz, 2H), 3.65 (t, J = 5.0 Hz, 2H), 3.97 (dd, J = 11.8 & 4.6 Hz, 2H), 4.13 (d, J = 4.4 Hz, 2H), 7.31 (t, J = 4.0 Hz, NH), 7.37-7.44 (m, 3H), 7.93-7.96 (m, 2H).
ESI/MS m/z: 471.0 (M+H)⁺
Yield: 33%

### Example 52

### 2-methyl-5-c-(2-(5-methyl-2-phenyloxazol-4-yl)ethyl)-N-(2-oxo-2-thiomorpholinoethyl)-1,3-dioxane-2-carboxamide

¹H NMR: 1.39 (q, J = 7.0 Hz, 2H), 1.50 (s, 3H), 2.03-2.08 (m, 1H), 2.31 (s, 3H), 2.41 (t, J = 7.6 Hz, 2H), 2.64-2.67 (m, 4H), 3.47 (t, J = 11.6 Hz, 2H), 3.69 (t, J = 4.8 Hz, 2H), 3.90 (t, J = 5.0 Hz, 2H), 3.98 (dd, J = 12.0 & 4.4 Hz, 2H), 4.12 (d, J = 4.4 Hz, 2H), 7.32 (bs, NH), 7.37-7.44 (m, 3H), 7.94-7.96 (m, 2H).
ESI/MS m/z : 474.0 (M+H)⁺
Yield: 70%

### Example 53

### 2-methyl-5-c-(2-(5-methyl-2-phenyloxazol-4-yl)ethyl)-N-(2-oxo-2-(piperidin-1-yl)ethyl)-1,3-dioxane-2-carboxamide

¹H NMR: 1.39 (q, J = 7.2 Hz, 2H), 1.49 (s, 3H), 1.55-1.69 (m, 6H), 2.02-2.08 (m, 1H), 2.30 (s, 3H), 2.41 (t, J = 7.8 Hz, 2H), 3.34 (t, J = 5.4 Hz, 2H), 3.48 (t, J = 11.6 Hz, 2H), 3.57 (t, J = 5.4 Hz, 2H), 3.97 (dd, J = 12.0 & 4.4 Hz, 2H), 4.12 (d, J = 4.0 Hz, 2H), 7.37-7.44 (m, 3H), 7.93-7.96 (m, 2H).
ESI/MS m/z : 456.0 (M+H)⁺
Yield: 85%

### Example 54

### 2-methyl-5-(2-(5-methyl-2-phenyloxazol-4-yl)ethyl)-N-(3-oxo-1-phenylbutan-2-yl)-1,3-dioxane-2-carboxamide

ESI/MS m/z : 477.4 (M+H)⁺
Yield: 28%

### Example 55

### 2-methyl-5-(2-(5-methyl-2-phenyloxazol-4-yl)ethyl)-N-(2-oxo-2-((3-oxo-1-phenylbutan-2-yl)amino)ethyl)-1,3-dioxane-2-carboxamide

ESI/MS m/z : 534.1 (M+H)⁺
Yield: 53%

### Example 56

### N-benzyloxy-2-methyl-5-((5-methyl-2-(p-tolyl)oxazol-4-yl)methyl)-1,3-dioxane-2-carboxamide

ESI/MS m/z : 459.1 (M+Na)⁺
Yield: 63%

### Example 57

### N-benzyloxy-2-methyl-5-c-((5-methyl-2-(p-tolyl)oxazol-4-yl)methyl)-1,3-dioxane-2-carboxam ide

¹H NMR: 1.43 (s, 3H), 2.19 (d, J = 7.2 Hz, 2H), 2.25 (s, 3H), 2.38 (s, 3H), 2.33-2.37 (m, 1H), 3.46 (t, J = 11.2 Hz, 2H), 3.91 (dd, J = 12 & 4.4 Hz, 2H), 4.98 (s, 2H), 7.21 (d, J = 8.0 Hz, 2H), 7.38-7.43 (m, 5H), 7.83 (d, J = 8.0 HHz, 2H), 8.68 (s, NH).
ESI/MS m/z : 437.1 (M+H)⁺
Yield: 68%

### Example 58

### N-benzyloxy-2-methyl-5-t-((5-methyl-2-(p-tolyl)oxazol-4-yl)methyl)-1,3-dioxane-2-carboxamide

¹H NMR: 1.49 (s, 3H), 1.84-1.86 (m, 1H), 2.32 (s, 3H), 2.38 (s, 3H), 2.78 (d, J = 7.6 Hz, 2H), 3.67 (d, J = 10.8 Hz, 2H), 3.85-3.88 (dd, J = 12 & 2.8 Hz, 2H), 4.99 (s, 2H), 7.22 (d, J = 8.0 Hz, 2H), 7.35-7.43 (m, 5H), 7.84 (d, J = 8.0 HHz, 2H), 8.65 (s, NH).
ESI/MS m/z : 437.1 (M+H)⁺
Yield: 55%

### Example 59

### N-hydroxy-2-methyl-5-((5-methyl-2-(p-tolyl)oxazol-4-yl)methyl)-1,3-dioxane-2-carboxamide

ESI/MS m/z : 368.9 (M+Na)⁺
Yield: 65%

### Example 60

### N-hydroxy-2-methyl-5-c-((5-methyl-2-(p-tolyl)oxazol-4-yl)methyl)-1,3-dioxane-2-carboxamide

¹H NMR(DMSO-D₆): 1.27 (s, 3H), 2.14-2.19 (m, 3H), 2.26 (s, 3H), 2.33 (s, 3H), 3.41 (t, J = 11.2 Hz, 2H), 3.77 (dd, J = 11.2 & 4.4 Hz, 2H), 7.29 (d, J = 8.0 Hz, 2H), 7.77 (d, J = 8.0 HHz, 2H).
ESI/MS m/z : 346.2 (M+H)⁺
Yield: 52%

### Example 61

### N-hydroxy-2-methyl-5-t-((5-methyl-2-(p-tolyl)oxazol-4-yl)methyl)-1,3-dioxane-2-carboxamide

¹H NMR: 1.52 (s, 3H), 1.94-1.95 (m, 1H), 2.34 (s, 3H), 2.40 (s, 3H), 2.78 (d, J = 7.6 Hz, 2H), 3.77 (d, J = 11.2 Hz, 2H), 3.96 (d, J = 10.8 Hz, 2H), 7.22 (d, J = 8.0 Hz, 2H), 7.84 (dd, J = 8.4 & 2.4 Hz, 2H), 8.96 (bs, NH).
ESI/MS m/z : 347.0 (M+H)⁺
Yield: 62%

### Example 62

### 2-methyl-5-((5-methyl-2-(p-tolyl)oxazol-4-yl)methyl)-N-((tetrahydro-2H-pyran-4-yl)methyl)-1,3-dioxane-2-carboxamide

ESI/MS m/z : 429.3 (M+H)⁺
Yield: 55%

### Example 63

### 2-methyl-5-c-((5-methyl-2-(p-tolyl)oxazol-4-yl)methyl)-N-((tetrahydro-2H-pyran-4-yl)methyl)-1,3-dioxane-2-carboxamide

¹H NMR: 1.29 -1.40 (m, 2H), 1.49 (s, 3H), 1.54-1.62 (m, 2H), 1.78-1.79 (m, 1H), 2.26-2.27 (m, 5H), 2.36-2.39 (m, 4H), 3.24 (t, J = 6.6 Hz, 2H), 3.37 (t, J = 11.8 Hz, 2H), 3.54 (t, J = 11 Hz, 2H), 3.96-4.03 (m, 4H), 6.45 (t, J = 6.0 Hz, NH), 7.23 (d, J = 8.0 Hz, 2H), 7.84 (d, J = 8.0 HHz, 2H).
ESI/MS m/z : 429.1 (M+H)⁺
Yield: 46%

### Example 64

### 2-methyl-5-t-((5-methyl-2-(p-tolyl)oxazol-4-yl)methyl)-N-((tetrahydro-2H-pyran-4-yl)methyl)-1,3-dioxane-2-carboxamide

¹H NMR: 1.08 -1.16 (m, 2H), 1.35 (s, 3H), 1.48-1.51 (m, 2H), 1.69-1.73 (m, 2H), 2.32 (s, 3H), 2.34 (s, 3H), 2.73 (d, J = 8.0 Hz, 2H), 2.97 (t, J = 6.6 Hz, 2H), 3.21 (t, J = 10.8 Hz, 2H), 3.62 (d, J = 10.8 Hz, 2H), 3.78-3.84 (m, 4H), 7.29 (d, J = 8.0 Hz, 2H), 7.77 (d, J = 8.4 HHz, 2H), 8.07 (t, J = 6.0 Hz, NH).
ESI/MS m/z : 429.2 (M+H)⁺
Yield: 43%

### Example 65

### (2-methyl-5-((5-methyl-2-(p-tolyl)oxazol-4-yl)methyl)-1,3-dioxan-2-yl)(morpholino)methanone

ESI/MS m/z : 401.2 (M+H)⁺
Yield: 46%

### Example 66

### (2-methyl-5-c-((5-methyl-2-(p-tolyl)oxazol-4-yl)methyl)-1,3-dioxan-2-yl)(morpholino)methanone

¹H NMR: 1.33 (s, 3H), 2.20-2.21 (m, 3H), 2.27 (s, 3H), 2.34 (s, 3H), 3.52-3.56 (m, 8H), 3.76-3.79 (m, 2H), 3.80-3.84 (dd, J = 11.2 Hz & 3.2 Hz, 2H), 7.29 (d, J = 8.0 Hz, 2H), 7.78 (d, J = 8.0 Hz, 2H).
ESI/MS m/z : 401.1 (M+H)⁺
Yield: 80%

### Example 67

### (2-methyl-5-t-((5-methyl-2-(p-tolyl)oxazol-4-yl)methyl)-1,3-dioxan-2-yl)(morpholino)methanone

¹H NMR (DMSO-D₆): 1.41 (s, 3H), 1.75-1.76 (m, 1H), 2.32 (s, 3H), 2.33 (s, 3H), 2.73 (d, J = 7.6 Hz, 2H), 3.50-3.58 (m, 6H), 3.64 (d, J = 10.8 Hz, 2H), 3.77-3.79 (m, 2H), 3.92 (dd, J = 11.6 2.4 Hz, 2H), 7.29 (d, J = 8.4 Hz, 2H), 7.77 (d, J = 8.4 Hz, 2H).
ESI/MS m/z : 401.1 (M+H)⁺
Yield: 73%

### Example 68

### 2-methyl-5-((5-methyl-2-(p-tolyl)oxazol-4-yl)methyl)-N-(2-morpholino-2-oxoethyl)-1,3-dioxane-2-carboxamide

ESI/MS m/z : 458.1 (M+H)⁺
Yield: 67%

### Example 69

### 2-methyl-5-c-((5-methyl-2-(p-tolyl)oxazol-4-yl)methyl)-N-(2-morpholino-2-oxoethyl)-1,3-dioxane-2-carboxamide

¹H NMR (DMSO-D₆): 1.30 (s, 3H), 2.14-2.19 (m, 3H), 2.27 (s, 3H), 2.31 (s, 3H); 3.40-3.43 (m, 4H), 3.53-3.64 (m, 6H), 3.79-3.82 (dd, J = 11.8 & 4.4 Hz, 2H), 3.96 (d, J = 5.6 Hz, 2H), 7.29 (d, J = 8.0 Hz, 2H), 7.78 (d, J = 8.0 , 2H), 7.93 (t, J = 5.6 Hz, NH).
ESI/MS m/z : 458.4 (M+H)⁺
Yield: 68%

### Example 70

2-methyl-5-*t*-((5-methyl-2-(p-tolyl)oxazol-4-yl)methyl)-N-(2-morpholino-2-oxoethyl)-1,3-dioxane-2-carboxamide
ESI/MS m/z : 458.2 (M+H)⁺
Yield: 59%

### Example 71

### N-(benzyloxy)-5-((2-(tert-butyl)-5-methyloxazol-4-yl)methyl)-2-methyl-1,3-dioxane-2-carboxamide

ESI/MS m/z : 403.2 (M+H)⁺
Yield: 54%

### Example 72

### 5-((2-(tert-butyl)-5-methyloxazol-4-yl)methyl)-N-hydroxy-2-methyl-1,3-dioxane-2-carboxam ide

ESI/MS m/z : 313.3 (M+H)⁺
Yield: 50%

### Example 73

### (5-((2-(tert-butyl)-5-methyloxazol-4-yl)methyl)-2-methyl-1,3-dioxan-2-yl)(morpholino)methanone

ESI/MS m/z : 367.2 (M+H)⁺
Yield: 38%

### Example 74

### 2-methyl-5-c-((5-methyl-2-(5-methylthiophen-2-yl)oxazol-4-yl)methyl)-N-((tetrahydro-2H-pyran-4-yl)methyl)-1,3-dioxane-2-carboxamide

¹H NMR (DMSO-D₆): 1.09-1.15 (m, 2H), 1.27 (s, 3H), 1.46-1.49 (m, 2H), 1.70-1.71 (m, 1H), 2.11-2.18 (m, 3H), 2.23 (s, 3H), 2.46 (s, 3H), 2.96 (t, J = 6.4 Hz, 2H), 3.18-3.24 (m, 2H), 3.35 (t, J = 11.2 Hz, 2H), 3.78-3.81 (m, 4H), 6.85 (dd, J = 3.6 & 0.8 Hz, 1H), 7.37 (d, J = 3.6 Hz, 1H), 8.03 (t, J = 6.0 Hz, NH).
ESI/MS m/z: 435.1 (M+H)⁺
Yield: 88%

### Example 75

### 2-methyl-5-t-((5-methyl-2-(5-methylthiophen-2-yl)oxazol-4-yl)methyl)-N-((tetrahydro-2H-pyran-4-yl)methyl)-1,3-dioxane-2-carboxamide

¹H NMR: 1.25-1.36 (m, 2H), 1.53 (s, 3H), 1.59-1.62 (m, 2H), 1.73-1.75 (m, 1H), 1.91-1.95 (m, 1H), 2.33 (s, 3H), 2.52 (s, 3H), 2.76 (d, J = 7.6 Hz, 2H), 3.21 (t, J = 6.6 Hz, 2H), 3.34-3.40 (m, 2H), 3.72-3.75 (dd, J = 11.8 & 2.6 Hz, 2H), 3.93-4.00 (m, 4H), 6.47 (t, J = 6.0 Hz, NH), 6.73 (dd, J = 2.2 & 1.0 Hz, 1H), 7.36 (d, J = 3.6 Hz, 1H).
ESI/MS m/z : 435.1 (M+H)⁺
Yield: 66%

### Example 76

### 2-methyl-5-c-((5-methyl-2-(5-methylthiophen-2-yl)oxazol-4-yl)methyl)-1,3-dioxan-2-yl)(morpholino)methanone

¹H NMR (DMSO-D₆): 1.33 (s, 3H), 2.16-2.17 (m, 3H), 2.24 (s, 3H), 3.27 (s, 3H), 3.50-3.57 (m, 8H), 3.76-3.82 (m, 4H), 6.85 (dd, J = 3.6 & 0.8 Hz, 1H), 7.37 (d, J = 3.6 Hz, 1H).
ESI/MS m/z : 407.0 (M+H)⁺
Yield: 92%

### Example 77

### 2-methyl-5-t-((5-methyl-2-(5-methylthiophen-2-yl)oxazol-4-yl)methyl)-1,3-dioxan-2-yl)(morpholino)methanone

¹H NMR (DMSO-D₆): 1.41 (s, 3H), 1.72-1.73 (m, 1H), 2.29 (s, 3H), 2.47 (s, 3H), 2.69 (d, J = 7.6 Hz, 2H), 3.51-3.64 (m, 8H), 3.77-3.82 (m, 2H), 3.92 (dd, J = 11.6 & 2.4 Hz, 2H), 6.85 (dd, J = 3.6 & 0.8 Hz, 1H), 7.37 (d, J = 3.6 Hz, 1H).
ESI/MS m/z : 407.0 (M+H)⁺
Yield: 75%

### Example 78

### N-benzyloxy-2-methyl-5-c-((5-methyl-2-(pyridin-4-yl)oxazol-4-yl)methyl)-1,3-dioxane-2-carboxamide

¹H NMR: 1.44 (s, 3H), 2.20 (d, J = 7.2 Hz, 2H), 2.29 (s, 3H), 2.31-2.34 (m, 1H), 3.45 (t, J = 11.2 Hz, 2H), 3.90 (dd, J = 12.0 & 4.4 Hz, 2H), 4.99 (s, 2H), 7.37-7.44 (m, 5H), 7.78 (dd, J = 4.4 & 1.6 Hz, 2H), 8.66 (dd, J = 4.6 & 1.4 Hz, 2H), 8.97 (s, NH).
ESI/MS m/z : 423.9 (M+H)⁺
Yield: 75%

### Example 79

### N-benzyloxy-2-methyl-5-t-((5-methyl-2-(pyridin-4-yl)oxazol-4-yl)methyl)-1,3-dioxane-2-carboxamide

¹H NMR: 1.49 (s, 3H), 1.81-1.86 (m, 1H), 2.37 (s, 3H), 2.81 (d, J = 7.6 Hz, 2H), 3.66 (d, J = 10.8 Hz, 2H), 3.87-3.90 (m, 2H), 5.00 (s, 2H), 7.33-7.43 (m, 5H), 7.78-7.80 (m, 2H), 8.68 (dd, J = 4.6 & 1.4 Hz, 2H).
ESI/MS m/z : 424.0 (M+H)⁺
Yield: 75%

### Example 80

### N-hydroxy-2-methyl-5-c-((5-methyl-2-(pyridin-4-yl)oxazol-4-yl)methyl)-1,3-dioxane-2-carboxamide

¹H NMR (DMSO-D₆): 1.28 (s, 3H), 2.12-2.20 (m, 1H), 2.23 (d, J = 7.2 Hz, 2H), 2.32 (s, 3H), 3.41 (t, J = 11.4 Hz, 2H), 3.78 (dd, J = 11.8 & 4.2 Hz, 2H), 7.79 (dd, J = 4.4 & 1.6 Hz, 2H), 8.69 (dd, J = 4.4 & 1.6 Hz, 2H).
ESI/MS m/z : 333.9 (M+H)⁺
Yield: 61%

### Example 81

### N-hydroxy-2-methyl-5-t-((5-methyl-2-(pyridin-4-yl)oxazol-4-yl)methyl)-1,3-dioxane-2-carboxamide

¹H NMR (DMSO-D₆): 1.36 (s, 3H), 1.74-1.75 (m, 1H), 2.37 (s, 3H), 2.77 (d, J = 7.6 Hz, 2H), 3.60 (d, J = 10.8 Hz, 2H), 3.84 (dd, J = 11.8 & 2.6 Hz, 2H), 7.78-7.80 (m, 2H), 8.68-8.70 (m, 2H).
ESI/MS m/z : 333.9 (M+H)⁺
Yield: 33%

### Example 82

### (2-methyl-5-c-((5-methyl-2-(pyridin-4-yl)oxazol-4-yl)methyl)-1,3-dioxan-2-yl)(morpholino)methanone

¹H NMR (DMSO-D₆): 1.34 (s, 3H), 2.20-2.26 (m, 3H), 2.33 (s, 3H), 3.50-3.57 (m, 8H), 3.76-3.77 (m, 2H), 3.82-3.86 (m, 2H), 7.79 (dd, J = 4.6 & 1.8 Hz, 2H), 8.69 (dd, J = 4.6 & 1.4 Hz, 2H).
ESI/MS m/z : 387.9 (M+H)⁺
Yield: 49%

### Example 83

### (2-methyl-5-t-((5-methyl-2-(pyridin-4-yl)oxazol-4-yl)methyl)-1,3-dioxan-2-yl)(morpholino)methanone

¹H NMR (DMSO-D₆): 1.42 (s, 3H), 1.75-1.78 (m, 1H), 2.37 (s, 3H), 2.78 (d, J = 7.6 Hz, 2H), 3.51-3.58 (m, 6H), 3.63 (d, J = 10.8 Hz, 2H), 3.76-3.78 (m, 2H), 3.93 (dd, J = 11.8 & 2.6 Hz, 2H), 7.78-7.80 (m, 2H), 8.68 (dd, J = 4.6 & 1.4 Hz, 2H).
ESI/MS m/z : 387.9 (M+H)⁺
Yield: 16%

### Example 84

### 2-methyl-5-c-((5-methyl-2-(pyridin-4-yl)oxazol-4-yl)methyl)-N-((tetrahydro-2H-pyran-4-yl)methyl)-1,3-dioxane-2-carboxamide

¹H NMR: 1.34-1.49 (m, 2H), 1.50 (s, 3H), 1.60-1.63 (m, 2H), 1.76-1.85 (m, 1H), 2.2-2.32 (m, 5H), 2.35-2.42 (m, 1H), 3.24 (t, J = 6.4 Hz, 2H), 3.35-3.42 (m, 2H), 3.52 (t, J = 11.0 Hz, 2H), 3.98-4.04 (m, 4H), 6.46 (t, J = 6.0 Hz, NH), 7.80 (dd, J = 4.8 & 1.6 Hz, 2H), 8.70 (dd, J = 4.4 & 1.6 Hz, 2H).
ESI/MS m/z : 416.0(M+H)⁺
Yield: 38%

### Example 85

### 2-methyl-5-t-((5-methyl-2-(pyridin-4-yl)oxazol-4-yl)methyl)-N-((tetrahydro-2H-pyran-4-yl)methyl)-1,3-dioxane-2-carboxamide

¹H NMR (DMSO-D₆): 1.09-1.13 (m, 2H), 1.35 (s, 3H), 1.48-1.51 (m, 2H), 1.70-1.74 (m, 2H), 2.37 (s, 3H), 2.77 (d, J = 7.6 Hz, 2H), 2.96 (t, J = 6.4 Hz, 2H), 3.18-3.24 (m, 2H), 3.61 (d, J = 10.8 Hz, 2H), 3.78-3.84 (m, 4H), 7.80 (dd, J = 4.4 & 1.6 Hz, 2H), 8.04 (t, J = 6.0 Hz, NH), 8.68 (m, 2H).
ESI/MS m/z : 416.0 (M+H)⁺
Yield: 27%

### Example 86

### 2-methyl-5-c-((5-methyl-2-(pyridin-4-yl)oxazol-4-yl)methyl)-N-(2-morpholino-2-oxoethyl)-1,3-dioxane-2-carboxamide

¹H NMR: 1.51 (s, 3H), 2.27 (d, J = 7.2 Hz, 2H), 2.31 (s, 3H), 2.38-2.43 (m, 1H), 3.43 (t, J = 4.8 Hz, 2H), 3.57-3.66 (m, 4H), 3.70-3.72 (m, 4H), 4.01 (dd, J = 12.0 & 4.4 Hz, 2H), 4.14 (d, J = 4.4 Hz, 2H), 7.31-7.33 (t, NH), 7.79 (dd, J = 4.8 & 1.4 Hz, 2H), 8.69 (d, J = 4.8 Hz, 2H).
ESI/MS m/z : 444.9 (M+H)⁺
Yield: 68%

### Example 87

### 2-methyl-5-t-((5-methyl-2-(pyridin-4-yl)oxazol-4-yl)methyl)-N-(2-morpholino-2-oxoethyl)-1,3-dioxane-2-carboxamide

¹H NMR: 1.56 (s, 3H), 1.88-1.92 (m, 1H), 2.40 (s, 3H), 2.88 (d, J = 7.6 Hz, 2H), 3.42 (t, J = 5.0 Hz, 2H), 3.63-3.65 (m, 2H), 3.69-3.71 (m, 4H), 3.76-3.78 (m, 2H), 4.02 (dd, J = 12.0 & 2.8 Hz, 2H), 4.13 (d, J = 4.4 Hz, 2H), 7.31-7.33 (t, NH), 7.79 (dd, J = 4.8 & 1.6 Hz, 2H), 8.68 (d, J = 6.0 Hz, 2H).
ESI/MS m/z : 445.2 (M+H)⁺
Yield: 63%

### Example 88

### 2-methyl-5-c-((5-methyl-2-(pyridin-4-yl)oxazol-4-yl)methyl)-N-(2-oxo-2-(((tetrahydro-2H-pyran-4-yl)methyl)amino)ethyl)-1,3-dioxane-2-carboxamide

¹H NMR: 1.30-1.37 (m, 2H), 1.51 (s, 3H), 1.59-1.63 (m, 2H), 1.71-1.79 (m, 1H), 2.28 (d, J = 7.6 Hz, 2H), 2.32 (s, 3H), 2.34-2.41 (m, 1H), 3.18 (t, J = 6.4 Hz, 2H), 3.33-3.39 (m, 2H), 3.55 (t, J = 10.8 Hz, 2H), 3.95-4.13 (m, 6H), 6.12 (bs, NH), 7.08 (t, J = 5.4 Hz, NH), 7.79 (d, J = 4.8 & 1.6 Hz, 2H), 8.69 (d, J = 4.8 & 1.6 Hz, 2H).
ESI/MS m/z : 473.3 (M+H)⁺
Yield: 53%

### Example 89

### 2-methyl-5-t-((5-methyl-2-(pyridin-4-yl)oxazol-4-yl)methyl)-N-(2-oxo-2-(((tetrahydro-2H-pyran-4-yl)methyl)amino)ethyl)-1,3-dioxane-2-carboxamide

¹H NMR: 1.27-1.35 (m, 2H), 1.55 (s, 3H), 1.57-1.61 (m, 2H), 1.69-1.77 (m, 1H), 1.92-1.97 (m, 1H), 2.40 (s, 3H), 2.84 (d, J = 7.6 Hz, 2H), 3.16 (t, J = 6.6 Hz, 2H), 3.31 (t, J = 11.6 Hz, 2H), 3.75-3.78 (m, 2H), 3.94-4.03 (m, 6H), 6.15 (m, NH), 7.08 (m, NH), 7.79 (d, J = 6.0 Hz, 2H), 8.69 (d, J = 3.6 Hz, 2H).
ESI/MS m/z : 473.2 (M+H)⁺
Yield: 63%

### Example 90

### N-benzyloxy-2-methyl-5-((4-methyl-2-(4-(trifluoromethyl)phenyl)thiazol-5-yl)methyl)-1,3-dioxane-2-carboxamide

ESI/MS m/z: 507.1 (M+H)⁺
Yield: 50%

### Example 91

### N-hydroxy-2-methyl-5-((4-methyl-2-(4-(trifluoromethyl)phenyl)thiazol-5-yl)methyl)-1,3-dioxane-2-carboxamide

ESI/MS m/z : 416.9 (M+H)⁺
Yield: 70%

### Example 92

### 1-(2-methyl-5-((4-methyl-2-(4-(trifluoromethyl)phenyl)thiazol-5-yl)methyl)-1,3-dioxan-2-yl)-N-((tetrahydro-2H-pyran-4-yl)methyl)methanamine

ESI/MS m/z : 499.6 (M+H)⁺
Yield: 99%

### Example 93

### (2-methyl-5-((4-methyl-2-(4-(trifluoromethyl)phenyl)thiazol-5-yl)methyl)-1,3-dioxan-2-yl)(morpholino)methanone

ESI/MS m/z : 471.5 (M+H)⁺
Yield: 51%

### Example 94

### (2-methyl-5-c-((4-methyl-2-(4-(trifluoromethyl)phenyl)thiazol-5-yl)methyl)-1,3-dioxan-2-yl)(morpholino)methanone

¹H NMR: 1.51 (s, 3H), 2.30-2.38 (m, 4H), 2.54 (d, J = 7.2 Hz, 2H), 3.59-3.67 (m, 4H), 3.73-3.75 (m, 4H), 3.87 (t, J = 4.2 Hz, 2H), 3.95-3.99 (dd, J = 12.0 & 4.4 Hz, 2H), 7.66 (d, J = 8.0 Hz, 2H), 7.97 (d, J = 8.0 Hz, 2H).
ESI/MS m/z : 471.0 (M+H)⁺
Yield: 36%

### Example 95

### (2-methyl-5-tran-((4-methyl-2-(4-(trifluoromethyl)phenyl)thiazol-5-yl)methyl)-1,3-dioxan-2-yl)(morpholino)methanone

¹H NMR: 1.57 (s, 3H), 1.63-1.67 (m, 1H), 2.47 (s, 3H), 3.23 (d, J = 8.0 Hz, 2H), 3.62-3.66 (m, 2H), 3.71-3.72 (m, 4H), 3.76-3.79 (dd, J = 12.0 & 4.0 Hz, 2H), 3.88 (t, J = 4.4 Hz, 2H), 4.09-4.12 (m, 2H), 7.66 (d, J = 8.4 Hz, 2H), 7.98 (d, J = 8.0 Hz, 2H).
ESI/MS m/z : 471.1 (M+H)⁺
Yield: 47%

### Example 96

### 5-c-(3-(9H-carbazol-9-yl)propyl)-N-hydroxy-2-methyl-1,3-dioxane-2-carboxamide

¹H NMR: 1.06-1.1 (m, 2H), 1.46 (s, 3H), 1.80-1.95 (m, 3H), 3.35 (t, J = 11.0 Hz, 2H), 3.85₋3.88 (m, 2H), 4.27 (t, J = 6.8 Hz, 2H), 7.21-7.26 (m, 2H), 7.32 (d, J = 8.4 Hz, 2H), 7.45-7.48 (m, 2H), 8.08-8.10 (m, 2H).
ESI/MS m/z : 368.9 (M+H)⁺
Yield: 37%

### Example 97

### N-hydroxy-5-c-(3-(10H-phenothiazin-10-yl)propyl)-2-methyl-1,3-dioxane-2-carboxamide

¹H NMR: 1.16-1.19 (m, 2H), 1.48 (s, 3H), 1.72-1.78 (m, 2H), 1.93-1.94 (m, 1H), 3.32 (t, J = 11.2 Hz, 2H), 3.81-3.89 (m, 4H), 6.80-6.86 (m, 2H), 6.90 (t, J = 7.6 Hz, 2H), 7.13-7.16 (m, 4H).
ESI/MS m/z : 401.0 (M+H)⁺
Yield: 40%

### Example 98

### N-(benzyloxy)-5-((3-(tert-butyl)-1-(p-tolyl)-1H-pyrazol-5-yl)methyl)-2-methyl-1,3-dioxane-2-carboxamide

ESI/MS m/z : 478.4 (M+H)⁺
Yield: 56%

### Example 99

### N-(hydroxy)-5-c-((3-(tert-butyl)-1-(p-tolyl)-1H-pyrazol-5-yl)methyl)-2-methyl-1,3-dioxane-2-carboxamide

¹H NMR: 1.31 (s, 9H), 1.46 (s, 3H), 2.14-2.19 (m, 1H), 2.37 (s, 2H), 2.39 (s, 3H), 3.35 (t, J = 11.0 Hz, 2H), 3.87 (dd, J = 12.0 & 4.0 Hz, 2H), 6.02 (s, 1H), 7.20-7.23 (m, 4H), 7.95 (bs, NH), 8.75 (bs, OH).
ESI/MS m/z : 387.9 (M+H)⁺
Yield: 10%

### Example 100

### N-(hydroxy)-5-t-((3-(tert-butyl)-1-(p-tolyl)-1H-pyrazol-5-yl)methyl)-2-methyl-1,3-dioxane-2-carboxamide

¹H NMR: 1.31 (s, 9H), 1.43 (s, 3H), 1.64-1.68 (m, 1H), 2.38 (s, 3H), 2.91 (d, J = 7.6 Hz, 2H), 3.65 (dd, J = 11.8 Hz, 2H), 3.86 (dd, J = 12.0 & 2.8 Hz, 2H), 6.04 (s, 1H), 7.21-7.29 (m, 4H).
ESI/MS m/z : 388.2 (M+H)⁺
Yield: 32%

### Example 101

### 5-((3-(tert-butyl)-1-(p-tolyl)-1H-pyrazol-5-yl)methyl)-2-methyl-N-((tetrahydro-2H-pyran-4-yl)methyl)-1,3-dioxane-2-carboxamide

ESI/MS m/z : 470.2 (M+H)⁺
Yield: 90%

### Example 102

### 5-((3-(tert-butyl)-1-(p-tolyl)-1H-pyrazol-5-yl)methyl)-2-methyl-N-(2-oxo-2-(((tetrahydro-2H-pyran-4-yl)methyl)amino)ethyl)-1,3-dioxane-2-carboxamide

ESI/MS m/z : 527.0 (M+H)⁺
Yield: 33%

### Example 103

### (5-((3-(tert-butyl)-]-(p-tolyl)-1H-pyrazol-5-yl)methyl)-2-methyl-1,3-dioxan-2-yl)(morpholino)methanone

ESI/MS m/z : 442.4 (M+H)⁺
Yield: 46%

### Example 104

### 5-((3-(tert-butyl)-1-(p-tolym)-1H-pyrazol-5-yl)methyl)-2-methyl-N-(2-morpholino-2-oxoethyl)-1,3-dioxane-2-carboxamide

ESI/MS m/z : 499.0 (M+H)⁺
Yield: 56%

### Example 105

(5-((3-(tert-butyl)-1-(p-tolyl)-1H-pyrazol-5-yl)methyl)-2-methyl-1,3-dioxan-2-yl)(4-methylpiperazin-1-yl)methanone

ESI/MS m/z : 455.0 (M+H)⁺
Yield: 50%

### Example 106

### N-(benzyloxy)-5-c-((2-(4-methoxyphenyl)-5-methyloxazol-4-yl)methyl)-2-methyl-1,3-dioxane-2-carboxamide

¹H NMR: 1.43 (s, 3H), 2.18 (d, J = 7.2 Hz, 2H), 2.24 (s, 3H), 2.33-2.38 (m, 1H), 3.46 (t, J = 11.2 Hz, 2H), 3.84 (s, 3H), 3.90-3.94 (dd, J = 11.8 & 4.8 Hz, 2H), 4.98 (s, 2H), 6.92 (d, J = 6.8 Hz, 2H), 7.35-7.43 (m, 5H), 7.87 (d, J = 6.8 Hz, 2H), 8.68 (s, 1H). ESI/MS m/z : 453.1 (M+H)⁺
Yield: 60%

### Example 107

### N-(benzyloxy)-5-t-((2-(4-methoxyphenyl)-5-methyloxazol-4-yl)methyl)-2-methyl-1,3-dioxane-2-carboxamide

¹H NMR: 1.48 (s, 3H), 1.82-1.87 (m, 1H), 2.31 (s, 3H), 2.77 (d, J = 7.6 Hz, 2H), 3.66-3.69 (dd, J = 12.2 & 2.0 Hz, 2H), 3.85 (s, 3H), 3.85-3.88 (dd, J = 12.2 & 2.8 Hz, 2H), 4.99 (s, 2H), 6.93 (d, J = 7.2 Hz, 2H), 7.35-7.43 (m, 5H), 7.88 (d, J = 6.8 Hz, 2H), 8.66 (s, NH).
ESI/MS m/z : 453.2 (M+H)⁺
Yield: 45%

### Example 108

### N-hydroxy-5-c-((2-(4-methoxyphenyl)-5-methyloxazol-4-yl)methyl)-2-methyl-1,3-dioxane-2-carboxamide

¹H NMR (DMSO-D₆): 1.28 (s, 3H), 1.12-2.18 (m, 3H), 2.26 (s, 3H), 3.41 (t, J = 5.6Hz, 2H), 3.78-3.82 (m, 5H), 7.03 (d, J = 6.8 Hz, 2H), 7.82 (d, J = 5.6 Hz, 2H), 8.40 (s, NH), 10.83 (s, OH).
ESI/MS m/z : 363.0 (M+H)⁺
Yield: 71%

### Example 109

### N-hydroxy-5-t-((2-(4-methoxyphenyl)-5-methyloxazol-4-yl)methyl)-2-methyl-1,3-dioxane-2-carboxamide

¹H NMR (DMSO-D₆): 1.35 (s, 3H), 1.73-1.74 (m, 1H), 2.30 (s, 3H), 2.71 (d, J = 7.6 Hz, 2H), 3.60 (d, J = 10.8 Hz, 2H), 3.79 (s, 3H), 3.83-3.86 (dd, J = 11.6 & 2.4 Hz, 2H), 7.02 (d, J = 7.2 Hz, 2H), 7.81 (d, J = 6.8 Hz, 2H), 8.83 (s, NH), 10.81 (s, OH).
ESI/MS m/z : 363.1 (M+H)⁺
Yield: 59%

### Example 110

### (5-c-((2-(4-methoxyphenyl)-5-methyloxazol-4-yl)methyl)-2-methyl-1,3-dioxan-2-yl)(morpholino)methanone

¹H NMR (DMSO-D₆): 1.33 (s, 3H), 2.19 (br S, 3H), 2.26 (s, 3H), 3.49-3.56 (m, 8H), 3.76 (br S, 2H), 3.80 (s, 3H), 3.82 (br S, 2H), 7.03 (d, J = 6.8 Hz, 2H), 7.82 (d, J = 6.8 Hz, 2H).
ESI/MS m/z : 417.1 (M+H)⁺
Yield: 84%

### Example 111

### (5-t-((2-(4-methoxyphenyl)-5-methyloxazol-4-yl)methyl)-2-methyl-1,3-dioxan-2-yl)(morpholino)methanone

¹H NMR (DMSO-D₆): 1.41 (s, 3H), 2.30 (s, 3H), 2.72 (d, J = 7.6 Hz, 2H), 3.51-3.56 (m, 6H), 3.64 (d, J = 10.8 Hz, 2H), 3.77-3.79 (m, 5H), 3.94 (d, J = 9.2 Hz, 2H), 7.02 (d, J = 6.8 Hz, 2H), 7.81 (d, J = 6.8 Hz, 2H).
ESI/MS m/z : 417.1 (M+H)⁺
Yield: 75%

### Example 112

### 5-c-((2-(4-methoxyphenyl)-5-methyloxazol-4-yl)methyl)-2-methyl-N-((tetrahydro-2H-pyran-4-yl)methyl)-1,3-dioxane-2-carboxamide

¹H NMR (DMSO-D₆): 1.08 -1.13 (m, 2H), 1.27 (s, 3H), 1.46-1.49 (m, 2H), 1.69 (m, 1H), 2.14-2.20 (m, 3H), 2.24 (s, 3H), 2.97 (t, J = 6.6 Hz, 2H), 3.20 (t, J = 11.4 Hz, 2H), 3.41 (t, J = 11 Hz, 2H), 3.77-3.83 (m, 7H), 7.04 (d, J = 6.8 Hz, 2H), 7.81 (d, J = 6.8 Hz, 2H), 8.05 (t, J = 6.0 Hz, 1H).
ESI/MS m/z : 445.2 (M+H)⁺
Yield: 59%

### Example 113

### 5-t-((2-(4-methoxyphenyl)-5-methyloxazol-4-yl)methyl)-2-methyl-N-((tetrahydro-2H-pyran-4-yl)methyl)-1,3-dioxane-2-carboxamide

¹H NMR (DMSO-D₆): 1.10 -1.14 (dd, J = 12.4 & 4.4 Hz, 2H), 1.36 (s, 3H), 1.49-1.52 (m, 2H), 1.71-1.74 (m, 1H), 2.32 (s, 3H), 2.72 (d, J = 7.2 Hz, 2H), 2.99 (t, J = 6.4 Hz, 2H), 3.22 (t, J = 10.8 Hz, 2H), 3.63 (d, J = 11.2 Hz, 2H), 3.81-3.85 (m, 7H), 7.04 (d, J = 8.8 Hz, 2H), 7.81 (d, J = 8.8 Hz, 2H), 8.05 (s, 1H).
ESI/MS m/z : 445.2 (M+H)⁺
Yield: 47%

### Example 114

### (5-c-((2-(4-methoxyphenyl)-5-methyloxazol-4-yl)methyl)-2-methyl-1,3-dioxan-2-yl)(4-methylpiperazin-1-yl)methanone

¹H NMR (DMSO-D₆): 1.32 (s, 3H), 2.15 (s, 3H), 2.19 (s, 3H), 2.25-2.28 (m, 7H), 3.50-3.53 (m, 4H), 3.73 (br S, 2H), 3.80 (s, 3H), 3.82 (br S, 2H), 7.03 (d, J = 7.2 Hz, 2H), 7.82 (d, J = 6.8 Hz, 2H).
ESI/MS m/z : 430.3(M+H)⁺
Yield: 95%

### Example 115

### (5-t-((2-(4-methoxyphenyl)-5-methyloxazol-4-yl)methyl)-2-methyl-1,3-dioxan-2-yl)(4-methylpiperazin-1-yl)methanone

¹H NMR (DMSO-D₆): 1.40 (s, 3H), 1.74 (br S, 1H), 2.20 (br S, 3H), 2.30 (s, 3H), 2.35 (br S, 4H), 2.72 (d, J = 7.6 Hz, 2H), 3.47 (br S, 2H), 3.64 (d, J = 10.8 Hz, 2H), 3.77-3.81 (m, 5H), 3.90-3.94 (dd, J= 11.8 Hz,2H), 7.03 (d, J = 6.8 Hz, 2H), 7.79-7.82 (m, 2H).
ESI/MS m/z : 430.1 (M+H)⁺
Yield: 89%

### Example 116

### N-(benzyloxy)-2-methyl-5-c-((5-methyl-2-(4-(trifluoromethyl)phenyl)oxazol-4-yl)methyl)-1,3-dioxane-2-carboxamide

¹H NMR: 1.39 (s, 3H), 2.20 (d, J = 7.2 Hz, 2H), 2.28 (s, 3H), 2.32-2.39 (m, 1H), 3.45 (t, J = 10.8 Hz, 2H), 3.91 (dd, J = 11.6 & 4.4 Hz, 2H), 4.99 (s, 2H), 7.35-7.44 (m. 5H), 7.66 (d, J = 8.4 Hz, 2H), 8.04 (d, J = 8.0 Hz, 2H), 8.69 (s, NH).
ESI/MS m/z : 490.9 (M+H)⁺
Yield: 59%

### Example 117

### N-(benzyloxy)-2-methyl-5-t-((5-methyl-2-(4-(trifluoromethyl)phenyl)oxazol-4-yl)methyl)-1,3-dioxane-2-carboxamide

¹H NMR: 1.49 (s, 3H), 1.82-1.87 (m, 1H), 2.36 (s, 3H), 2.80 (d, J = 8.0 Hz, 2H), 3.66 (dd, J = 12.8 & 1.6 Hz, 2H), 3.87 (dd, J = 11.6 & 2.8 Hz, 2H), 5.00 (s, 2H), 7.31-7.43 (m. 5H), 7.67 (d, J = 8.0 Hz, 2H), 8.05 (d, J = 8.0 Hz, 2H), 8.67 (s, NH).
ESI/MS m/z : 490.1 (M+H)⁺
Yield: 58%

### Example 118

### N-hydroxy-2-methyl-5-c-((5-methyl-2-(4-(trifluoromethyl)phenyl)oxazol-4-yl)methyl)-1,3-dioxane-2-carboxamide

¹H NMR: 1.52 (s, 3H), 2.28 (d, J = 7.6 Hz, 2H), 2.30 (s, 3H), 2.35-2.43 (m, 1H), 3.54 (t, J = 11.2 Hz, 2H), 4.01 (dd, J = 11.8 & 4.2 Hz, 2H), 7.67 (d, J = 8.0 Hz, 2H), 8.05 (d, J = 8.0 Hz, 2H).
ESI/MS m/z : 401.3 (M+H)⁺
Yield: 52%

### Example 119

### N-hydroxy-2-methyl-5-t-((5-methyl-2-(4-(trifluoromethyl)phenyl)oxazol-4-yl)methyl)-1,3-dioxane-2-carboxamide

ESI/MS m/z : 401.1 (M+H)⁺
Yield: 59%

### Example 120

### (2-methyl-5-c-((5-methyl-2-(4-(trifluoromethyl)phenyl)oxazol-4-yl)methyl)-1,3-dioxan-2-yl)(morpholino)methanone

¹H NMR: 1.50 (s, 3H), 2.22 (d, *J*= 7.2 Hz, 2H), 2.31 (s, 3H), 2.43-2.52 (m, 1H), 3.61-3.78 (m, 8H), 3.90 (t, *J*= 4.6 Hz, 2H), 3.91 (dd, *J*= 12.0 & 4.8 Hz, 2H), 7.68 (d, *J*= 8.4 Hz, 2H), 8.06 (d, *J*= 8.0 Hz, 2H).
ESI/MS m/z : 455.2 (M+H)⁺
Yield: 60%

### Example 121

### (2-methyl-5-t-((5-methyl-2-(4-(trifluoromethyl)phenyl)oxazol-4-yl)methyl)-1,3-dioxan-2-yl)(morpholino)methanone

¹H NMR: 1.55 (s, 3H), 1.86-1.90 (m, 1H), 2.40 (s, 3H), 2.90 (d, J = 7.6 Hz, 2H), 3.64-3.67 (m, 2H), 3.71-3.77 (m, 6H), 3.88-3.91 (m, 2H), 4.07-4.11 (m, 2H), 7.67 (d, J = 8.4 Hz, 2H), 8.06 (d, J = 8.0 Hz, 2H)
ESI/MS m/z : 455.1 (M+H)⁺
Yield: 90%

### Example 122

### 2-methyl-5-c-((5-methyl-2-(4-(trifluoromethyl)phenyl)oxazol-4-yl)methyl)-N-((tetrahydro-2H-pyran-4-yl)methyl)-1,3-dioxane-2-carboxamide

¹H NMR: 1.26-1.39 (m, 2H), 1.49 (s, 3H), 1.59-1.62 (m, 2H), 1.75-1.83 (m, 1H), 2.27 (d, J = 7.2 Hz, 2H), 2.30 (s, 3H), 2.34-2.44 (m, 1H), 3.23 (t, J = 6.6 Hz, 2H), 3.34 (t, J = 11.8 Hz, 2H), 3.52 (t, J = 10.6 Hz, 2H), 3.97-4.04 (m, 4H), 6.43 (t, J = 6.0 Hz, NH), 7.67 (d, J = 8.4 Hz, 2H), 8.05 (d, J = 8.4 Hz, 2H)
ESI/MS m/z : 483.1 (M+H)⁺
Yield: 80%

### Example 123

### 2-methyl-5-t-((5-methyl-2-(4-(trifluoromethyl)phenyl)oxazol-4-yl)methyl)-N-((tetrahydro-2H-pyran-4-yl)methyl)-1,3-dioxane-2-carboxamide

¹H NMR: 1.25-1.39 (m, 2H), 1.54 (s, 3H), 1.58-1.62 (m, 2H), 1.73-1.83 (m, 1H), 1.92-1.98 (m, 1H), 2.39 (s, 3H), 2.82 (d, J = 8.0 Hz, 2H), 3.22 (t, J = 6.4 Hz, 2H), 3.41 (t, J = 11.8 Hz, 2H), 3.75-3.78 (m, 2H), 3.96-4.00 (m, 2H), 6.45 (t, J = 6.2 Hz, NH), 7.67 (d, J = 8.4 Hz, 2H), 8.05 (d, J = 8.4 Hz, 2H)
ESI/MS m/z : 483.2 (M+H)⁺
Yield: 58%

In an embodiment is provided a method of identifying small molecules as inhibitors of PCSK9 through in-silico modeling. For identifying molecules which can bind to the PCSK9, an insilico mutant of PCSK9 was generated without prodomain which was subsequently taken for docking simulations. It was surprisingly found that small molecules which bind to the catalytic site as provided below, shows relevant in-vitro and in-vivo effects of PCSK9 inhibition. Nomenclature including numbering of amino acids is as per 3BPS crystal structure of PCSK9 obtained from protein databank (www.pdb.org).

### Amino acid sequence of the catalytic site of PCSK9:

ASP 186 THR 187 LYS 222 HIS 224 HIS 226 LEU 230 VAL 252 LEU 253 ASN 254 GLN 256 GLY 257 LYS 258 GLY 259 THR 260 VAL 261 SER 262 THR 264 LEU 287 PRO 288 LEU 289 ALA 290 GLY 291 GLY 292 LEU 297 ALA 314 ALA 315 ASN 317 PHE 318 TYR 325 SER 326 PRO 327 SER 386 GLN 387

In a preferred embodiment, the small molecules which bind to one or more of the amino acids comprising the catalytic triad HIS 226 SER 386 ASP 186 alone or in combination with other amino acids from the catalytic site.

### Docking studies:

PCSK9 binding molecules were identified using *in silico* docking studies using PCSK9 protein crystal structure (3BPS) crystallized with EGF-A domain of LDL-R *(*PNAS, 105 (6), 1820-1825, 2008), pro-domain and CRD obtained from publicly available database protein databank (PDB). Computationally we have generated a mutant of PCSK9 without pro-domain, wherein the active site consists of but not restricted to the following amino acids:
ASP 186 THR 187 LYS 222 HIS 226 LEU 230 VAL 252 LEU 253 ASN 254 GLN 256 GLY 257 LYS 258 GLY 259 THR 260 VAL 261 SER 262 THR 264 LEU 287 PRO 288 LEU 289 ALA 290 GLY 291 GLY 292 LEU 297 ALA 314 ALA 315 ASN 317 PHE 318 TYR 325 SER 326 PRO 327 SER 386 GLN 387

PCSK9 protein (3BPS.pdb) was prepared for docking studies using Protein Preparation Wizard tool (Schrödinger Suite 2010 Protein Preparation Wizard; *Epik version 2.1;* **Schrödinger, LLC: New York, NY**, **2010;** *Impact version 5.6;* **Schrodinger, LLC: New York, NY, 2010**; *Prime version 2.2;* **Schrodinger, LLC**: New York, NY, 2010) implemented in Maestro. All the bond orders were assigned, and the hydrogen atoms were added to the protein as per the standard protocol. Geometry of the compounds to be docked were subsequently optimized using the LigPrep version 2.6 *(LigPrep 2.6;* **Schrodinger, LLC: New York, NY, 2010**). Docking studies were carried out for these compounds using Glide version 5.6 (*Glide 5.6:* **Schrodinger, LLC: New York, NY**, **2010**), the automated docking program implemented in the Schrodinger package.

One of the representative examples of the small molecule docked as per the above protocol is provided in figure 1.

### Docking Results:

| **Example No.** | **G-score** | **H-bonds** |
|---|---|---|
| 1 | -6.40 | Gly 259 C=O...HN of hydroxylamine |
| 4 | -6.98 | Asp 186 C=O...HO of hydroxyl amine |
| | | Asp 186 O...HN of hydroxylamine |
| 14 | -6.03 | Lys 222 NH...O=C attached to Piperidine |
| 19 | -7.00 | Gly 291 NH...O ofdioxane |
| 37 | -6.14 | Gly 257 C=O...HN of amide attached to dioxane |
| 40 | -5.21 | Lys 222 NH...O=C attached to dioxane |
| 47 | -7.35 | Lys 222 NH...O=C attached to dioxane |

The above in-silico results can be further supported by x-ray diffraction of the complex of the compound co-crystallized with the PCSK9 protein. Further support can be obtained by selectively mutating the amino acids in the catalytic site and testing the compounds through Elisa using this mutant protein.

### Biological studies:

The compounds of the present invention lowered LDL, triglyceride and total cholesterol. This was demonstrated by *in vitro* as well as *in vivo* animal experiments.

### A) Demonstration of in vitro efficacy of compounds

The PCSK9-LDLR *in vitro* Binding Assay is a quantitative solid phase binding assay between PCSK9 and recombinant LDLR. Plates were pre-coated with a recombinant LDLR-AB domain, which binds PCSK9. Test compound at different concentration was added to the PCSK9 and added to LDLR immobilized on the wells. The amount of bound PCSK9 is measured by binding it with biotinylated anti-His-tag monoclonal antibody, followed by binding with horseradish peroxidase conjugated streptavidin substrate. The color was quantified by ELISA reader at 450 nM which reflects the relative amount of PCSK9 that binds to LDLR in presence and absence of the inhibitor. EC₅₀ values were calculated by nonlinear regression analysis using graph pad prism software. Each concentration point represents values in duplicates.

| **Example No.** | **Concentration (µM)** | % **Inhibition PCSK9** |
|---|---|---|
| 1 | 5 | 25 |
| | 10 | 30 |
| | 100 | 32 |
| 4 | 10 | 35 |
| | 100 | 42 |
| 6 | 5 | 10 |
| | 10 | 11 |
| | 100 | 22 |
| 7 | 1 | 11 |
| | 10 | 12 |
| | 100 | 20 |
| 8 | 1 | 18 |
| | 10 | 23 |
| | 100 | 30 |
| 9 | 10 | 15 |
| | 100 | 12 |
| 11 | 5 | 20 |
| | 10 | 30 |
| | 100 | 44 |
| 12 | 10 | 19 |
| | 100 | 18 |
| 14 | 10 | 41 |
| | 100 | 5 |
| 15 | 10 | 11 |
| | 100 | 4 |
| 16 | 100 | 12 |
| 17 | 100 | 15 |
| 18 | 1 | 40 |
| | 100 | 39 |
| 19 | 10 | 23 |
| | 100 | 25 |
| 22 | 10 | 25 |
| | 100 | 17 |
| 23 | 10 | 21 |
| | 100 | 22 |
| 25 | 10 | 15 |
| | 100 | 16 |
| 29 | 10 | 8 |
| | 100 | 12 |
| 34 | 1 | 11 |
| | 10 | 12 |
| | 100 | 15 |
| 35 | 1 | 18 |
| | 10 | 19 |
| | 100 | 19 |
| 36 | 1 | 10 |
| | 100 | 24 |
| 37 | 1 | 24 |
| | 10 | 15 |
| | 100 | 20 |
| 38 | 10 | 14 |
| | 100 | 21 |
| 40 | 1 | 20 |
| | 10 | 25 |
| | 100 | 11 |
| 41 | 10 | 12 |
| | 100 | 19 |
| 43 | 10 | 8 |
| | 100 | 10 |
| 44 | 10 | 14 |
| | 100 | 25 |
| 45 | 10 | 11 |
| | 100 | 18 |
| 46 | 10 | 12 |
| | 100 | 18 |
| 47 | 1 | 38 |
| | 10 | 38 |
| | 100 | 42 |
| 49 | 100 | 17 |
| 51 | 10 | 10 |
| | 100 | 35 |
| 52 | 10 | 20 |
| | 100 | 32 |
| 54 | 10 | 21 |
| | 100 | 29 |
| 55 | 10 | 23 |
| | 100 | 30 |
| 56 | 5 | 6 |
| | 10 | 17 |
| 59 | 1 | 26 |
| | 10 | 27 |
| | 100 | 61 |
| 60 | 1 | 28 |
| | 10 | 26 |
| | 100 | 70 |
| 61 | 10 | 21 |
| | 100 | 32 |
| 62 | 0.1 | 17 |
| | 1 | 21 |
| | 10 | 14 |
| | 100 | 41 |
| 63 | 1 | 39 |
| | 10 | 40 |
| | 100 | 51 |
| 64 | 0.1 | 20 |
| | 1 | 23 |
| | 10 | 25 |
| | 100 | 49 |
| 65 | 1 | 11 |
| | 10 | 26 |
| | 100 | 35 |
| 66 | 1 | 23 |
| | 10 | 25 |
| | 100 | 31 |
| 67 | 1 | 3 |
| | 10 | 21 |
| | 100 | 25 |
| 72 | 5 | 11 |
| | 110 | 20 |
| | 100 | 25 |
| 73 | 1 | 25 |
| | 10 | 27 |
| | 100 | 37 |
| 74 | 1 | 4 |
| | 10 | 15 |
| | 100 | 27 |
| 75 | 1 | 15 |
| | 10 | 11 |
| | 100 | 20 |
| 76 | 1 | 6 |
| | 10 | 17 |
| | 100 | 27 |
| 77 | 10 | 14 |
| | 100 | 16 |
| 80 | 1 | 32 |
| | 10 | 44 |
| | 100 | 34 |
| 81 | 1 | 7 |
| | 10 | 12 |
| | 100 | 23 |
| 82 | 1 | 13 |
| | 10 | 20 |
| | 100 | 42 |
| 83 | 1 | 20 |
| | 10 | 25 |
| | 100 | 42 |
| 84 | 1 | 25 |
| | 10 | 29 |
| | 100 | 65 |
| 85 | 1 | 22 |
| | 10 | 30 |
| | 100 | 44 |
| 86 | 10 | 11 |
| | 100 | 15 |
| 87 | 10 | 12 |
| | 100 | 7 |
| 88 | 10 | 10 |
| | 100 | 15 |
| 89 | 10 | 7 |
| | 100 | 10 |
| 91 | 1 | 8 |
| | 10 | 12 |
| | 100 | 32 |
| 92 | 1 | 11 |
| | 10 | 26 |
| | 100 | 40 |
| 93 | 1 | 20 |
| | 10 | 16 |
| | 100 | 49 |
| 96 | 10 | 10 |
| 99 | 10 | 5 |
| | 100 | 32 |
| 105 | 10 | 10 |
| | 100 | 40 |

### B) SPR Binding studies:

### Immobilization protocol:

Instrumentation: BIACORE3000 from GE Life Sciences was used and the Interaction analysis was performed at 25°C.

Buffers: phosphate buffer pH 7.4 supplied by Biacore supplemented with 0.01% dimethyl sulfoxide (DMSO) was used as running buffer during the assay.

Immobilization of Ligand: Sensor chip CM5 was used for analysis. The sensor chip consists of a carboxymethyl-modified dextran polymer linked to a gold-covered glass support. The ligand was diluted to 10 micro gram / ml in 10mM sodium acetate, pH5.0 and immobilized to the sensor chip, using amine coupling. Running buffer without DMSO was used during immobilization. The surface was activated by injecting a solution containing 0.2M N-ethyl-N'-dimethyl aminopropyl-carbodiimide (EDC) and 50mM N-hydroxy succinimide (NHS). The system was set for Aim for immobilization of about 1000 RU and the surface was blocked by injecting 1Mehanolamine at pH 8.5. Then the surface was washed with 50mM NaOH by injecting two 30 second-pulses to remove off non covalently bound ligand and to stabilize the baseline (automated procedure). Activated dextran was used as a reference surface. To ensure highest sensitivity the SPR Detector response was normalized before running the assay. By calibrating the detector at various light intensities under Conditions of total internal reflection (automated procedure). Solvent correction for DMSO was also carried to minimize the error in data.

Assay Design: A binding assay was set up for different compounds at three different concentrations (10nM, 100nM and 1 micro mole), and were injected over the reference and Ligand flow cells at a flow rate of 30 micro liter / min. Each cycle consisted of a 1-min waiting period for monitoring of the baseline stability and 3 minute injection of compound with a 5 minute Undisturbed dissociation phase, and the surface was regenerated with 50 mM NaoH solution. Compound responses in the reference flow cell were subtracted with that of ligand and a solvent correction run was carried in between and the binding potential of the compounds was reported as relative response units (RU).

| **Example No.** | **Relative response (RU)** |
|---|---|
| 4 | 5 13 |
| 6 | 2 65 |
| 14 | 15 81 |
| 19 | 14 1 |
| 26 | 10 3 |
| 34 | 1 95 |
| 47 | 2 64 |
| 59 | 1 36 |
| 64 | 3 48 |
| 73 | 24 |
| 80 | 2 64 |
| 84 | 2 23 |
| 85 | 17.29 |
| 86 | 14.95 |

### C) Demonstration of in vivo efficacy of compounds

### i) LDL-C lowering activity in LPS induced dyslipidemia model C57 mice

Male C57 mice of 7-10 week age are grouped based on non-fasting LDL-C levels, on next day these mice were administered with Vehicle or test compounds (i.p. or oral) and 30 min after compound administration lipolysaccharide (LPS) was administered. After LPS administration all animals were kept on fasting for 24 hours, after that animals were bled and LDL-C was measured. The percent change in LDL-C in test compound group Vs Vehicle group was calculated.

| **Example No** | **% Change in LDL-C** |
|---|---|
| 4 | -29.4 ± 4.1 |
| 8 | -33.5 ± 5.4 |
| 12 | -40 ± 10.0 |
| 37 | -35.4 ± 8.3 |
| 40 | -34.1 ± 5.7 |
| 63 | -9.0 ± 10.0 |
| 84 | -25.8 ± 8.6 |
| 91 | -37.4 ± 8.8 |
| 92 | -35.8 ± 10.5 |
| 93 | -46.0 ± 6.2 |

### ii) Lipid lowering effect in HF-HC diet fed Hamster model

In this experiment, Syrian golden hamsters which were kept on high fat -high cholesterol (HF-HC) fructose diet for 2 weeks were divided into various groups based on LDL-C and Total cholesterol levels. The treatment was given by oral gavages once daily for 14 days. The blood was collected by retro-orbital sinus puncture method under light ether anesthesia on day 0 (pretreatment), and day 14 of the treatment for lipid levels measurements. The percent change in LDL-C in compound group Vs Vehicle group was calculated.

| **Example No.** | **% Change Vs Vehicle Control** | | | |
|---|---|---|---|---|
| | **LDL-C** | **TG** | TC | **HDL-C** |
| 4 | -24 | -35 | -27 | -25 |
| 7 | -29.3 | -26.7 | -29.6 | -20.1 |
| 34 | -38.2 | 2.2 | -32.6 | -10.3 |

### iii) LDL-C lowering activity- in high fat diet C57 mice

The *in-vivo* LDL-c lowering for test compound was tested in C57 mice which were kept on high fat diet for 4 weeks and the blood was collected by retro-orbital sinus puncture method under light ether anesthesia on day 0 (pretreatment), animal are grouped based on LDL-C levels, after that 4-6 week treatment with vehicle or test compound orally once a day was given. On completion of treatment on day 28 day and or on day 42 of the treatment the blood was collected for LDL-C levels measurement. The percent change in LDL-C in test compound group Vs Vehicle group was calculated.

| **Example No** | **% Change Vs Vehicle Control** | | | |
|---|---|---|---|---|
| | **LDL-C** | **TG** | **TC** | **HDL-C** |
| 4 | -24 | -35 | -14 | -5 |

## Claims

1. Compounds of the general formula (I), their tautomeric forms, their stereoisomers, regioisomers, their pharmaceutically acceptable salts, and pharmaceutical compositions containing them 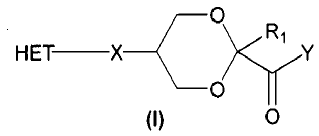 wherein 'HET' represents an optionally substituted heteroaryl or a heterocyclic group; 'X' represents the groups selected from (CH₂)ₙ, O(CH₂)ₙ, S(CH₂)ₙ, SO(CH₂)ₙ, SO₂(CH₂)ₙ or NR₂(CH₂)ₙ wherein R₂ represents H, (C₁-C₆) linear or branched alkyl or a suitable cycloalkyl group, wherein either the alkyl or cycloalkyl group may be further substituted; n = 0 to 3; 'R₁' represents linear or branched (C₁-C₆) alkyl, cycloalkyl, aryl, groups;
'Y' represents
i) the group NR₃R₄, wherein 'R₃' represents (C₁-C₆) linear or branched alkyl, cycloalkyl, cycloalkylalkyl, aryl, heteroaryl, heterocyclyl, arylalkyl, heteroarylalkyl, heterocyclylalkyl, hydroxy, alkoxy, cycloalkoxy, cycloalkylalkoxy, aryloxy, arylalkoxy, heteroaryloxy, heteroarylalkoxy, heterocyclyloxy or heterocyclylalkoxy groups, each of which may be further substituted & 'R₄' represents H, C₍₁₋₆₎ linear or branched alkyl, cycloalkyl, cycloalkylalkyl, aryl, heteroaryl, heterocyclyl, arylalkyl, heteroarylalkyl or heterocyclylalkyl groups, each of which may be further substituted;
ii) the groups 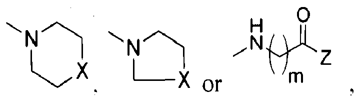 wherein 'P' represents CH₂, S, SO, SO₂, O, CO or NR₅ wherein R₅ represents H, (C₁-C₆) linear or branched alkyl or cycloalkyl groups; 'm' represents integers from 1 to 3; and 'Z' represents either NR₃R₄ or the groups 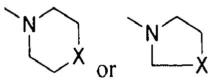 wherein 'R₃', 'R₄' and 'P' are as defined earlier.

2. The compounds as claimed in claim 1 wherein the substituents on 'HET' represents one or more groups selected from hydrogen or optionally substituted groups selected from (C₁-C₆) linear or branched alkyl, aryl, heteroaryl, cycloalkyl or a heterocyclyl group.

3. The compounds as claimed in claim 1 wherein the substituents on 'HET' are selected from (C₁-C₆) linear or branched alkyl, aryl or heteroaryl groups, each of which may be further substituted.

4. The compounds as claimed in claim 1 wherein when the groups representing 'HET' is further substituted, the substituents are selected from halogen, alkyl, haloalkyl, alkoxy, sulfanyl derivative, sulfinyl derivatives, sulfonyl derivatives, sulfonyloxy groups.

5. The compounds of formula (I) wherein the groups representing 'HET' is selected from 'HET' are selected from aziridinyl, azetidinyl, pyrrolidinyl, imidazolidinyl, piperidinyl, piperazinyl, 2-oxopiperidinyl, 4-oxopiperidinyl, 2-oxopiperazinyl, 3-oxopiperazinyl, morpholinyl, thiomorpholinyl, 2-oxomorpholinyl, azepinyl, diazepinyl, oxapinyl, thiazepinyl, oxazolidinyl, thiazolidinyl, dihydrothiophene, dihydropyran, dihydrofuran, dihydrothiazole, benzopyranyl, benzopyranonyl, benzodihydrofuranyl, benzodihydrothienyl, pyrazolopyrimidonyl, azaquinazolinoyl, thienopyrimidonyl, quinazolonyl, pyrimidonyl, benzoxazinyl, benzoxazinonyl, benzothiazinyl, benzothiazinonyl, thieno piperidinyl, pyridyl, thienyl, furyl, pyrrolyl, oxazolyl, thiazolyl, isothiazolyl, imidazolyl, isoxazolyl, oxadiazolyl, thiadiazolyl, triazolyl, tetrazolyl, benzofuranyl, benzothienyl, indolinyl, indolyl, azaindolyl, azaindolinyl, pyrazolopyrimidinyl, azaquinazolinyl, pyridofuranyl, pyridothienyl, thienopyrimidyl, quinolinyl, pyrimidinyl, pyrazolyl, quinazolinyl, pyridazinyl, triazinyl, benzimidazolyl, benzotriazolyl, phthalazynil, naphthylidinyl, purinyl, carbazolyl, phenothiazinyl, phenoxazinyl, benzoxazolyl, benzothiazolyl groups.

6. The compounds of formula (I) selected from
N-(benzyloxy)-2-methyl-5-((5-methyl-2-phenyloxazol-4-yl)methyl)-1,3-dioxane-2-carboxamide;
N-(benzyloxy)-2-methyl-5-*c*-((5-methyl-2-phenyloxazol-4-yl)methyl)-1,3-dioxane-2-carboxamide;
N-(benzyloxy)-2-methyl-5-*t*-((5-methyl-2-phenyloxazol-4-yl)methyl)-1,3-dioxane-2-carboxamide;
N-hydroxy-2-methyl-5-((5-methyl-2-phenyloxazol-4-yl)methyl)-1,3-dioxane-2-carboxamide;
5-*c*-(3-(9H-carbazol-9-yl)propyl)-N-hydroxy-2-methyl-1,3-dioxane-2-carboxamide;
N-hydroxy-2-methyl-5-*c*-((5-methyl-2-phenyloxazol-4-yl)methyl)-1,3-dioxane-2-carboxam ide;
N-hydroxy-2-methyl-5-*t*-((5-methyl-2-phenyloxazol-4-yl)methyl)-1,3-dioxane-2-carboxam ide;
2-methyl-5-((5-methyl-2-phenyloxazol-4-yl)methyl)-N-((tetrahydro-2H-pyran-4-yl)methyl)-1,3-dioxane-2-carboxamide;
2-methyl-5-*c*-((5-methyl-2-phenyloxazol-4-yl)methyl)-N-((tetrahydro-2H-pyran-4-yl)methyl)-1,3-dioxane-2-carboxamide;
2-methyl-5-*t*-((5-methyl-2-phenyloxazol-4-yl)methyl)-N-((tetrahydro-2H-pyran-4-yl)methyl)-1,3-dioxane-2-carboxamide;
(2-methyl-5-((5-methyl-2-phenyloxazol-4-yl)methyl)-1,3-dioxan-2-yl)(morpholino)methanone;
(2-methyl-5-*c*-((5-methyl-2-phenyloxazol-4-yl)methyl)-1,3-dioxan-2-yl)(morpholino)methanone;
(2-methyl-5-*t*-((5-methyl-2-phenyloxazol-4-yl)methyl)-1,3-dioxan-2-yl)(morpholino)methanone;
(2-methyl-5-*c*-((5-methyl-2-phenyloxazol-4-yl)methyl)-1,3-dioxan-2-yl)(4-methylpiperazin-1-yl)methanone;
(2-methyl-5-*c*-((5-methyl-2-phenyloxazol-4-yl)methyl)-1,3-dioxan-2-yl)(piperidin-1-yl)methanone;
(2-methyl-5-*c*-((5-methyl-2-phenyloxazol-4-yl)methyl)-1,3-dioxan-2-yl)(thiomorpholino)methanone;
(1,1-dioxidothiomorpholino)(2-methyl-5-*c*-((5-methyl-2-phenyloxazol-4-yl)methyl)-1,3-dioxan-2-yl)methanone;
2-methyl-5-((5-methyl-2-phenyloxazol-4-yl)methyl)-N-(2-morpholino-2-oxoethyl)-1,3-dioxane-2-carboxamide;
2-methyl-5-*c*-((5-methyl-2-phenyloxazol-4-yl)methyl)-N-(2-morpholino-2-oxoethyl)-1,3-dioxane-2-carboxamide;
2-methyl-5-*t*-((5-methyl-2-phenyloxazol-4-yl)methyl)-N-(2-morpholino-2-oxoethyl)-1,3-dioxane-2-carboxamide;
2-methyl-5-*c*-((5-methyl-2-phenyloxazol-4-yl)methyl)-N-(2-(4-methylpiperazin-1-yl)-2-oxoethyl)-1,3-dioxane-2-carboxamide;
2-methyl-5-*c*-((5-methyl-2-phenyloxazol-4-yl)methyl)-N-(2-oxo-2-(piperidin-1-yl)ethyl)-1,3-dioxane-2-carboxamide;
2-methyl-5-*c*-((5-methyl-2-phenyloxazol-4-yl)methyl)-N-(2-oxo-2-thiomorpholinoethyl)-1,3-dioxane-2-carboxamide;
N-(2-(1,1-dioxidothiomorpholino)-2-oxoethyl)-2-methyl-5-*c*-((5-methyl-2-phenyloxazol-4-yl)methyl)-1,3-dioxane-2-carboxamide;
2-methyl-5-*c*-((5-methyl-2-phenyloxazol-4-yl)methyl)-N-(2-oxo-2-(((tetrahydro-2H-pyran-4-yl)methyl)amino)ethyl)-1,3-dioxane-2-carboxamide;
2-methyl-5-*c*-((5-methyl-2-phenyloxazol-4-yl)methyl)-N-(3-morpholino-3-oxopropyl)-1,3-dioxane-2-carboxamide;
2-methyl-5-*c*-((5-methyl-2-phenyloxazol-4-yl)methyl)-N-(3-oxo-3-(((tetrahydro-2H-pyran-4-yl)methyl)amino)propyl)-1,3-dioxane-2-carboxamide;
2-methyl-5-*c*-((5-methyl-2-phenyloxazol-4-yl)methyl)-N-(3-oxo-3-thiomorpholinopropyl)-1,3-dioxane-2-carboxamide;
N-(3-(1,1-dioxidothiomorpholino)-3-oxopropyl)-2-methyl-5-*c*-((5-methyl-2-phenyloxazol-4-yl)methyl)-1,3-dioxane-2-carboxamide;
2-methyl-5-*c*-((5-methyl-2-phenyloxazol-4-yl)methyl)-N-(3-oxo-3-(piperidin-1-yl)propyl)-1,3-dioxane-2-carboxamide;
2-methyl-5-*c*-((5-methyl-2-phenyloxazol-4-yl)methyl)-N-(3-(4-methylpiperazin-1-yl)-3-oxopropyl)-1,3-dioxane-2-carboxamide;
2-methyl-5-((5-methyl-2-phenyloxazol-4-yl)methyl)-N-(3-oxo-1-phenylbutan-2-yl)-1,3-dioxane-2-carboxamide;
N-benzyloxy-2-methyl-5-(2-(5-methyl-2-phenyloxazol-4-yl)ethyl)-1,3-dioxane-2-carboxamide;
N-hydroxy-2-methyl-5-(2-(5-methyl-2-phenyloxazol-4-yl)ethyl)-1,3-dioxane-2-carboxamide;
N-hydroxy-2-methyl-5-*c*-(2-(5-methyl-2-phenyloxazol-4-yl)ethyl)-1,3-dioxane-2-carboxamide;
N-hydroxy-2-methyl-5-*t*-(2-(5-methyl-2-phenyloxazol-4-yl)ethyl)-1,3-dioxane-2-carboxamide;
2-methyl-5-(2-(5-methyl-2-phenyloxazol-4-yl)ethyl)-N-((tetrahydro-2H-pyran-4-yl)methyl)-1,3-dioxane-2-carboxamide;
2-methyl-5-*c*-(2-(5-methyl-2-phenyloxazol-4-yl)ethyl)-N-((tetrahydro-2H-pyran-4-yl)methyl)-1,3-dioxane-2-carboxamide;
2-methyl-5-*t*-(2-(5-methyl-2-phenyloxazol-4-yl)ethyl)-N-((tetrahydro-2H-pyran-4-yl)methyl)-1,3-dioxane-2-carboxamide;
(2-methyl-5-(2-(5-methyl-2-phenyloxazol-4-yl)ethyl)-1,3-dioxan-2-yl)(morpholino)methanone;
(2-methyl-5-*c*-(2-(5-methyl-2-phenyloxazol-4-yl)ethyl)-1,3-dioxan-2-yl)(morpholino)methanone;
(2-methyl-5-*t*-(2-(5-methyl-2-phenyloxazol-4-yl)ethyl)-1,3-dioxan-2-yl)(morpholino)methanone;
(2-methyl-5-*c*-(2-(5-methyl-2-phenyloxazol-4-yl)ethyl)-1,3-dioxan-2-yl)(4-methylpiperazin-1-yl)methanone;
(2-methyl-5-*c*-(2-(S-methyl-2-phenyloxazol-4-yl)ethyl)-1,3-dioxan-2-yl)(piperidin-1-yl)methanone;
(2-methyl-5-*c*-(2-(5-methyl-2-phenyloxazol-4-yl)ethyl)-1,3-dioxan-2-yl)(thiomorpholino)methanone;
(1,1-dioxidothiomorpholino)(2-methyl-5-*c*-(2-(5-methyl-2-phenyloxazol-4-yl)ethyl)-1,3-dioxan-2-yl)methanone;
2-methyl-5-(2-(5-methyl-2-phenyloxazol-4-yl)ethyl)-N-(2-morpholino-2-oxoethyl)-1,3-dioxane-2-carboxamide;
2-methyl-5-c-(2-(5-methyl-2-phenyloxazol-4-yl)ethyl)-N-(2-morpholino-2-oxoethyl)-1,3-dioxane-2-carboxamide;
2-methyl-5-*t*-(2-(5-methyl-2-phenyloxazol-4-yl)ethyl)-N-(2-morpholino-2-oxoethyl)-1,3-dioxane-2-carboxamide;
2-methyl-5-c-(2-(5-methyl-2-phenyloxazol-4-yl)ethyl)-N-(2-oxo-2-(((tetrahydro-2H-pyran-4-yl)methyl)amino)ethyl)-1,3-dioxane-2-carboxamide;
2-methyl-5-c-(2-(5-methyl-2-phenyloxazol-4-yl)ethyl)-N-(2-(4-methylpiperazin-1-yl)-2-oxoethyl)-1,3-dioxane-2-carboxamide;
2-methyl-5-c-(2-(5-methyl-2-phenyloxazol-4-yl)ethyl)-N-(2-oxo-2-thiomorpholinoethyl)-1,3-dioxane-2-carboxam ide;
2-methyl-5-*c*-(2-(5-methyl-2-phenyloxazol-4-yl)ethyl)-N-(2-oxo-2-(piperidin-1-yl)ethyl)-1,3-dioxane-2-carboxamide;
2-methyl-5-(2-(5-methyl-2-phenyloxazol-4-yl)ethyl)-N-(3-oxo-1-phenylbutan-2-yl)-1,3-dioxane-2-carboxamide;
2-methyl-5-(2-(5-methyl-2-phenyloxazol-4-yl)ethyl)-N-(2-oxo-2-((3-oxo-1-phenylbutan-2-yl)amino)ethyl)-1,3-dioxane-2-carboxamide;
N-benzyloxy-2-methyl-5-((5-methyl-2-(p-tolyl)oxazol-4-yl)methyl)-1,3-dioxane-2-carboxam ide;
N-benzyloxy-2-methyl-5-*c*-((5-methyl-2-(p-tolyl)oxazol-4-yl)methyl)-1,3-dioxane-2-carboxamide;
N-benzyloxy-2-methyl-5-*t*-((5-methyl-2-(p-tolyl)oxazol-4-yl)methyl)-1,3-dioxane-2-carboxamide;
N-hydroxy-2-methyl-5-((5-methyl-2-(p-tolyl)oxazol-4-yl)methyl)-1,3-dioxane-2-carboxamide;
N-hydroxy-2-methyl-5-*c*-((5-methyl-2-(p-tolyl)oxazol-4-yl)methyl)-1,3-dioxane-2-carboxamide;
N-hydroxy-2-methyl-5-*t*-((5-methyl-2-(p-tolyl)oxazol-4-yl)methyl)-1,3-dioxane-2-carboxamide;
2-methyl-5-((5-methyl-2-(p-tolyl)oxazol-4-yl)methyl)-N-((tetrahydro-2H-pyran-4-yl)methyl)-1,3-dioxane-2-carboxamide;
2-methyl-5-*c*-((5-methyl-2-(p-tolyl)oxazol-4-yl)methyl)-N-((tetrahydro-2H-pyran-4-yl)methyl)-1,3-dioxane-2-carboxamide;
2-methyl-5-*t*-((5-methyl-2-(p-tolyl)oxazol-4-yl)methyl)-N-((tetrahydro-2H-pyran-4-yl)methyl)-1,3-dioxane-2-carboxamide;
(2-methyl-5-((5-methyl-2-(p-tolyl)oxazol-4-yl)methyl)-1,3-dioxan-2-yl)(morpholino)methanone;
(2-methyl-5-*c*-((5-methyl-2-(p-tolyl)oxazol-4-yl)methyl)-1,3-dioxan-2-yl)(morpholino)methanone;
(2-methyl-5-*t*-((5-methyl-2-(p-tolyl)oxazol-4-yl)methyl)-1,3-dioxan-2-yl)(morpholino)methanone;
2-methyl-5-((5-methyl-2-(p-tolyl)oxazol-4-yl)methyl)-N-(2-morpholino-2-oxoethyl)-1,3-dioxane-2-carboxamide;
2-methyl-5-*c*-((5-methyl-2-(p-tolyl)oxazol-4-yl)methyl)-N-(2-morpholino-2-oxoethyl)-1,3-dioxane-2-carboxamide;
2-methyl-5-*t*-((5-methyl-2-(p-tolyl)oxazol-4-yl)methyl)-N-(2-morpholino-2-oxoethyl)-1,3-dioxane-2-carboxamide;
N-(benzyloxy)-5-((2-(tert-butyl)-5-methyloxazol-4-yl)methyl)-2-methyl-1,3-dioxane-2-carboxam ide;
5-((2-(tert-butyl)-5-methyloxazol-4-yl)methyl)-N-hydroxy-2-methyl-1,3-dioxane-2-carboxamide;
(5-((2-(tert-butyl)-5-methyloxazol-4-yl)methyl)-2-methyl-1,3-dioxan-2-yl)(morpholino)methanone;
2-methyl-5-*c*-((5-methyl-2-(5-methylthiophen-2-yl)oxazol-4-yl)methyl)-N-((tetrahydro-2H-pyran-4-yl)methyl)-1,3-dioxane-2-carboxamide;
2-methyl-5-*t*-((5-methyl-2-(5-methylthiophen-2-yl)oxazol-4-yl)methyl)-N-((tetrahydro-2H-pyran-4-yl)methyl)-1,3-dioxane-2-carboxamide;
2-methyl-5-*c*-((5-methyl-2-(5-methylthiophen-2-yl)oxazol-4-yl)methyl)-1,3-dioxan-2-yl)(morpholino)methanone;
2-methyl-5-*t*-((5-methyl-2-(5-methylthiophen-2-yl)oxazol-4-yl)methyl)-1,3-dioxan-2-yl)(morpholino)methanone;
N-benzyloxy-2-methyl-5-*c*-((5-methyl-2-(pyridin-4-yl)oxazol-4-yl)methyl)-1,3-dioxane-2-carboxamide;
N-benzyloxy-2-methyl-5-*t*-((5-methyl-2-(pyridin-4-yl)oxazol-4-yl)methyl)-1,3-dioxane-2-carboxamide;
N-hydroxy-2-methyl-5-*c*-((5-methyl-2-(pyridin-4-yl)oxazol-4-yl)methyl)-1,3-dioxane-2-carboxamide;
N-hydroxy-2-methyl-5-*t*-((5-methyl-2-(pyridin-4-yl)oxazol-4-yl)methyl)-1,3-dioxane-2-carboxamide;
(2-methyl-5-*c*-((5-methyl-2-(pyridin-4-yl)oxazol-4-yl)methyl)-1,3-dioxan-2-yl)(morpholino)methanone;
(2-methyl-5-*t*-((5-methyl-2-(pyridin-4-yl)oxazol-4-yl)methyl)-1,3-dioxan-2-yl)(morpholino)methanone;
2-methyl-5-*c*-((5-methyl-2-(pyridin-4-yl)oxazol-4-yl)methyl)-N-((tetrahydro-2H-pyran-4-yl)methyl)-1,3-dioxane-2-carboxamide;
2-methyl-5-*t*-((5-methyl-2-(pyridin-4-yl)oxazol-4-yl)methyl)-N-((tetrahydro-2H-pyran-4-yl)methyl)-1,3-dioxane-2-carboxamide;
2-methyl-5-*c*-((5-methyl-2-(pyridin-4-yl)oxazol-4-yl)methyl)-N-(2-morpholino-2-oxoethyl)-1,3-dioxane-2-carboxamide;
2-methyl-5-*t*-((5-methyl-2-(pyridin-4-yl)oxazol-4-yl)methyl)-N-(2-morpholino-2-oxoethyl)-1,3-dioxane-2-carboxamide;
2-methyl-5-*c*-((5-methyl-2-(pyridin-4-yl)oxazol-4-yl)methyl)-N-(2-oxo-2-(((tetrahydro-2H-pyran-4-yl)methyl)amino)ethyl)-1,3-dioxane-2-carboxamide;
2-methyl-5-*t*-((5-methyl-2-(pyridin-4-yl)oxazol-4-yl)methyl)-N-(2-oxo-2-(((tetrahydro-2H-pyran-4-yl)methyl)amino)ethyl)-1,3-dioxane-2-carboxamide;
N-benzyloxy-2-methyl-5-((4-methyl-2-(4-(trifluoromethyl)phenyl)thiazol-5-yl)methyl)-1,3-dioxane-2-carboxamide;
N-hydroxy-2-methyl-5-((4-methyl-2-(4-(trifluoromethyl)phenyl)thiazol-5-yl)methyl)-1,3-dioxane-2-carboxaimide;
1-(2-methyl-5-((4-methyl-2-(4-(trifluoromethyl)phenyl)thiazol-5-yl)methyl)-1,3-dioxan-2-yl)-N-((tetrahydro-2H-pyran-4-yl)methyl)methanamine;
(2-methyl-5-((4-methyl-2-(4-(trifluoromethyl)phenyl)thiazol-5-yl)methyl)-1,3-dioxan-2-yl)(morpholino)methanone;
(2-methyl-5-*c*-((4-methyl-2-(4-(trifluoromethyl)phenyl)thiazol-5-yl)methyl)-1,3-dioxan-2-yl)(morpholino)methanone;
(2-methyl-5-*t*-((4-methyl-2-(4-(trifluoromethyl)phenyl)thiazol-5-yl)methyl)-1,3-dioxan-2-yl)(morpholino)methanone;
5-*c*-(3-(9H-carbazol-9-yl)propyl)-N-hydroxy-2-methyl-1,3-dioxane-2-carboxamide; N-hydroxy-5-*c*-(3-(10H-phenothiazin-10-yl)propyl)-2-methyl-1,3-dioxane-2-carboxamide;
N-(benzyloxy)-5-((3-(tert-butyl)-1-(p-tolyl)-1H-pyrazol-5-yl)methyl)-2-methyl-1,3-dioxane-2-carboxamide;
N-(hydroxy)-5-*c*-((3-(tert-butyl)-1-(p-tolyl)-1H-pyrazol-5-yl)methyl)-2-methyl-1,3-dioxane-2-carboxamide;
N-(hydroxy)-5-*t*-((3-(tert-butyl)-1-(p-tolyl)-1H-pyrazol-5-yl)methyl)-2-methyl-1,3-dioxane-2-carboxamide;
5-((3-(tert-butyl)-1-(p-tolyl)-1H-pyrazol-5-yl)methyl)-2-methyl-N-((tetrahydro-2H-pyran-4-yl)methyl)-1,3-dioxane-2-carboxamide;
5-((3-(tert-butyl)-1-(p-tolyl)-1H-pyrazol-5-yl)methyl)-2-methyl-N-(2-oxo-2-(((tetrahydro-2H-pyran-4-yl)methyl)amino)ethyl)-1,3-dioxane-2-carboxamide;
(5-((3-(tert-butyl)-1-(p-tolyl)-1H-pyrazol-5-yl)methyl)-2-methyl-1,3-dioxan-2-yl)(morpholino)methanone;
5-((3-(tert-butyl)-1-(p-tolyl)-1H-pyrazol-5-yl)methyl)-2-methyl-N-(2-morpholino-2-oxoethyl)-1,3-dioxane-2-carboxamide;
(5-((3-(tert-butyl)-1-(p-tolyl)-1H-pyrazol-5-yl)methyl)-2-methyl-1,3-dioxan-2-yl)(4-methylpiperazin-1-yl)methanone;
N-(benzyloxy)-5-*c*-((2-(4-methoxyphenyl)-5-methyloxazol-4-yl)methyl)-2-methyl-1,3-dioxane-2-carboxamide;
N-(benzyloxy)-5-*t*-((2-(4-methoxyphenyl)-5-methyloxazol-4-yl)methyl)-2-methyl-1,3-dioxane-2-carboxamide;
N-hydroxy-5-*c*-((2-(4-methoxyphenyl)-5-methyloxazol-4-yl)methyl)-2-methyl-1,3-dioxane-2-carboxamide;
N-hydroxy-5-*t*-((2-(4-methoxyphenyl)-5-methyloxazol-4-yl)methyl)-2-methyl-1,3-dioxane-2-carboxamide;
(5-*c*-((2-(4-methoxyphenyl)-5-methyloxazol-4-yl)methyl)-2-methyl-1,3-dioxan-2-yl)(morpholino)methanone;
(5-*t*-((2-(4-methoxyphenyl)-5-methyloxazol-4-yl)methyl)-2-methyl-1,3-dioxan-2-yl)(morpholino)methanone;
5-*c*-((2-(4-methoxyphenyl)-5-methyloxazol-4-yl)methyl)-2-methyl-N-((tetrahydro-2H-pyran-4-yl)methyl)-1,3-dioxane-2-carboxamide;
5-*t*-((2-(4-methoxyphenyl)-5-methyloxazol-4-yl)methyl)-2-methyl-N-((tetrahydro-2H-pyran-4-yl)methyl)-1,3-dioxane-2-carboxamide;
(5-*c*-((2-(4-methoxyphenyl)-5-methyloxazol-4-yl)methyl)-2-methyl-1,3-dioxan-2-yl)(4-methylpiperazin-1-yl)methanone;
(5-*t*-((2-(4-methoxyphenyl)-5-methyloxazol-4-yl)methyl)-2-methyl-1,3-dioxan-2-yl)(4-methylpiperazin-1-yl)methanone;
N-(benzyloxy)-2-methyl-5-*c*-((5-methyl-2-(4-(trifluoromethyl)phenyl)oxazol-4-yl)methyl)-1,3-dioxane-2-carboxamide;
N-(benzyloxy)-2-methyl-5-*t*-((5-methyl-2-(4-(trifluoromethyl)phenyl)oxazol-4-yl)methyl)-1,3-dioxane-2-carboxamide;
N-hydroxy-2-methyl-5-*c*-((5-methyl-2-(4-(trifluoromethyl)phenyl)oxazol-4-yl)methyl)-1,3-dioxane-2-carboxamide;
N-hydroxy-2-methyl-5-*t*-((5-methyl-2-(4-(trifluoromethyl)phenyl)oxazol-4-yl)methyl)-1,3-dioxane-2-carboxamide;
(2-methyl-5-*c*-((5-methyl-2-(4-(trifluoromethyl)phenyl)oxazol-4-yl)methyl)-1,3-dioxan-2-yl)(morpholino)methanone;
(2-methyl-5-*t*-((5-methyl-2-(4-(trifluoromethyl)phenyl)oxazol-4-yl)methyl)-1,3-dioxan-2-yl)(morpholino)methanone;
2-methyl-5-*c*-((5-methyl-2-(4-(trifluoromethyl)phenyl)oxazol-4-yl)methyl)-N-((tetrahydro-2H-pyran-4-yl)methyl)-1,3-dioxane-2-carboxamide;
2-methyl-5-*t*-((5-methyl-2-(4-(trifluoromethyl)phenyl)oxazol-4-yl)methyl)-N-((tetrahydro-2H-pyran-4-yl)methyl)-1,3-dioxane-2-carboxamide.

7. A pharmaceutical composition comprising a therapeutically effective amount of a compound of formula (I) as claimed in any of the preceding claims and a pharmaceutically acceptable carrier diluent or excipients.

8. A pharmaceutical composition according to claim 7 which is used for the treatment of dyslipidemia and related diseases.

9. A method of treating disorders caused by dyslipidemia and related diseases comprising administering to a patient in need thereof an effective amount of a compound of formula (I) according to any of the preceding claims or its pharmaceutical composition according to any of the preceding claims.

10. The use of a compound of formula (I) or its pharmaceutical composition according to any of the preceding claims for the manufacture of a medicament for the treatment of dyslipidemia and related diseases.

11. A medicine for the treatment of disorders caused by dyslipidemia and related diseases which comprises administering therapeutically effective amount of compound of formula (I) or its pharmaceutical composition as defined in any of the preceding claims to a patient or subject in need thereof.

12. A method of selecting small molecule inhibitors of PCSK9 by identifying molecules which bind to the catalytic site of the PCSK9, and subsequently checking the binding potential of the identified molecules to the PCSK9 protein.

13. The method as claimed in claim 12 wherein the molecules bind to one or more of the amino acids comprising the catalytic triad HIS 226 SER 386 ASP 186 alone or in combination with other amino acids from the catalytic site.

14. Small molecules which bind to the catalytic site of the PCSK9, as inhibitors of PCSK9.

15. Small molecules inhibitors of PCSK9 which bind to the catalytic site of the PCSK9 protein.
